# EUROPEAN PATENT APPLICATION

(11) **EP 3 708 162 A1**
(43) Date of publication of application: **16.09.2020**
(21) Application number: 19382179.0
(22) Date of filing: 12.03.2019
(51) Int. Cl.: A61K 31/407, A61K 9/00, A61P 25/28, A61P 39/06

(54) **MACROCYCLES WITH ANTIOXIDANT AND NEUROPROTECTIVE ACTIVITIES**

(71) Applicant: Instituto Biomar S.A., 24009 Armunia, León (ES)
(72) Inventor: FERNÁNDEZ MEDARDE, Antonio, E-24009 Armunia, León (ES); VINUESA NAVARRO, María de los Ángeles, E-24009 Armunia, León (ES); SÁNCHEZ LÓPEZ, José María, E-24009 Armunia, León (ES); BOLAÑOS HERNÁNDEZ, Juan Pedro, E-37007 Salamanca (ES); ALMEIDA PARRA, María de los Ángeles, E-37007 Salamanca (ES); FERNANDEZ SANCHEZ, Emilio, E-37007 Salamanca (ES)
(74) Representative: ABG Intellectual Property Law, S.L.

(57) **Abstract**

Compounds of general formula 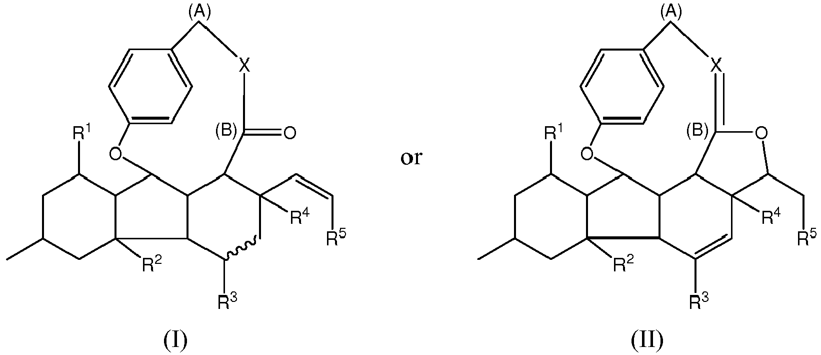 wherein
the wavy bond ∼ is either a double bond or the epoxide, 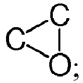 or pharmaceutically acceptable salts, solvates, tautomers, or stereoisomers thereof, for use in the treatment of diseases associated with oxidative stress.

## Description

### TECHNICAL FIELD

The present invention relates to macrocycles, pharmaceutical compositions containing them and their use as antioxidant and neuroprotective products.

### BACKGROUND OF THE INVENTION

Histopathological examination of biopsies or necropsies has revealed that a considerable number of human chronic and acute diseases are associated with biochemical signs of oxidative stress. Oxidative stress defines an abnormal condition in which cells cannot efficiently eliminate the endogenous formation of reactive oxygen and nitrogen species, collectively named as RONS (Bolaños JP, et al. Adv. Drug Deliv. Rev. 61:1299-1315, 2009). RONS (including one or more of the following species: hydrogen peroxide or H₂O₂, superoxide anion or O₂^{•-} , nitric oxide or ^{•}NO, peroxynitrite anion or ONOO⁻, hydroxyl radical or ^{•}OH, amongst others) are physiologically synthesized molecules that play important cell signaling functions; however, RONS are intrinsically unstable and rapidly disintegrate by chemical reaction with themselves, oxygen, or cellular constituents such as lipids, nucleic acids or proteins. Moreover, disintegration of RONS actually takes place mainly by the action of specific enzymes, generally named as antioxidant enzymes. These include several families of proteins, including glutathione peroxidases, glutathione reductase, superoxide dismutases, catalase, peroxyredoxins, thioredoxins, etc, which are ubiquitously distributed in tissues and in cells. Some of these antioxidant enzymes need cofactors to be fully functional, the most important of which are glutathione and NADPH. Of these, glutathione is also considered as an antioxidant, since it can also react directly with RONS, hence contributing to their removal, although the overall contribution of this scavenging effect of glutathione is minimal. In addition, the enzymes that are responsible for the biosynthesis or regeneration of these cofactors are considered as antioxidant enzymes too, and include glutamate-cysteine ligase (the enzyme that catalyzes the rate-limiting step in glutathione biosynthesis), and glucose-6-phosphate dehydrogenase (the first and rate-limiting enzyme of the pentose-phosphate pathway, which regenerates NADPH from its oxidized form, NADP).

The formation of RONS takes place ubiquitously, although the major sources are complexes I and III of the mitochondrial respiratory chain (which form O₂^{•-}), plasma membrane-bound NADPH oxidase (which forms O₂^{•-}), nitric oxide synthases (which forms ^{•}NO), superoxide dismutase (which forms H₂O₂ from O₂^{•-}), as well as chemical reactions of O₂^{•-} with cations (copper or iron) forming ^{•}OH. RONS formation by the mitochondrial complex I can be strongly stimulated by the action of rotenone. Cells are normally and continuously producing and degrading RONS in a balanced manner that prevents from the accumulation of RONS and therefore avoids their potentially cellular damaging effects. However, under certain pathophysiological situations leading to either excess RONS formation or impaired antioxidant-mediated RONS elimination, oxidative stress occurs. Due to the overall and indiscriminate damage to cellular nucleic acids, lipids and proteins, oxidative stress causes cytotoxicity, tissue and organ dysfunction and eventually death. There are a lot of human diseases that are associated with this oxidative stress, as neurodegenerative diseases such as Parkinson's disease, Alzheimer's disease, senile dementia, Huntington's disease, multiple sclerosis or amyotrophic lateral sclerosis, stroke, mitochondriopathies with neuromuscular affection, cardiovascular disease, pancreatitis, and cirrhosis.

Despite the profound knowledge in cellular RONS metabolism (i.e., RONS formation and removal) and its highly significant association with human diseases, to date there is no antioxidant pharmacological strategy that has been proven to be effective (i.e., protective or repairing) in clinical trials (Kamat CD, et all. J. Alzheimers Dis 15:473-493, 2008). In this context, the proposed pharmacological strategies used up to date have been based on the capacity of the drugs to scavenge, i.e. remove and inactivate by direct reaction, RONS, hence theoretically contributing to preventing and/or blocking oxidative stress. Perplexingly, the studies carried out in certain human disease animal models (in rodents) associated with oxidative stress, have yielded very promising results with some antioxidant pharmacological strategies that thereafter failed in the clinical trial. Reasons for this discrepancy are unknown.

The design and development of small molecules with antioxidant and neuroprotective properties is therefore an attractive approach for the development of new therapeutic agents.

Pyrrocidines A, B and C have been disclosed (Wicklow DT, et al., Phytopathology, 99: 109-15, 2009; Haiyin HH, et al., Tetrahedron Lett., 43: 1633-6, 2002; Wicklow DT, et al., Mycol. Res. 109: 610-8, 2005; Yoshihito S et al., Bioorg. Med. Chem. Lett. 18: 6050-6053, 2008; Ear A. et al., Organic & biomolecular chemistry 13.12: 3662-3666, 2015; US7129266B2; Casella, Thiago M., et al. Phytochemistry 96: 370-377, 2013) as antimicrobial, cytotoxic and apoptosis inductor compounds.

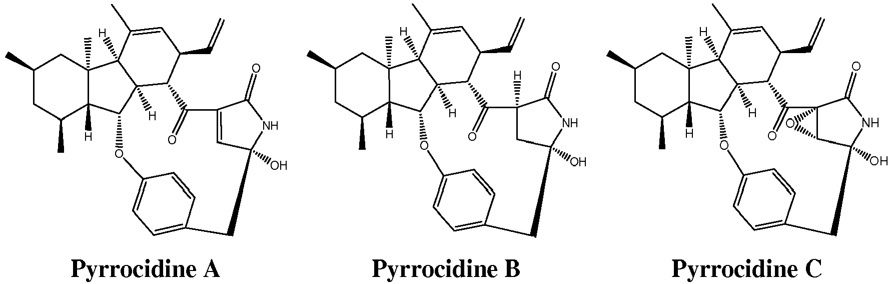

Hirsutellone A-E have been disclosed (Masahiko I, et all., Tetrahedron 61: 5577-83, 2005; US20060122252) as antibacterial compounds against *Mycobacterium tuberculosis.*

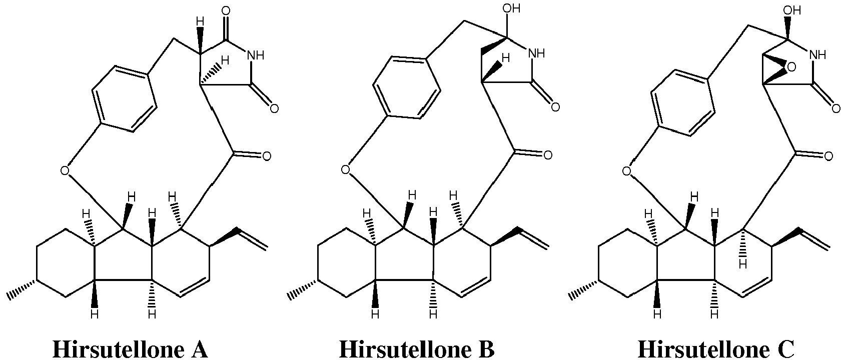

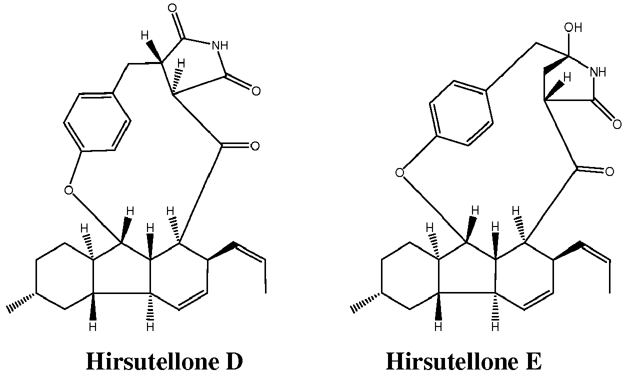

GKK1032A1 and A2 have been disclosed (JP2001247574) as antimicrobial and antitumoral compounds. GKK1032 has been disclosed (Pastre R et al. Quimica Nova, 30: 1867-71, 2007) as an antimicrobial compound.

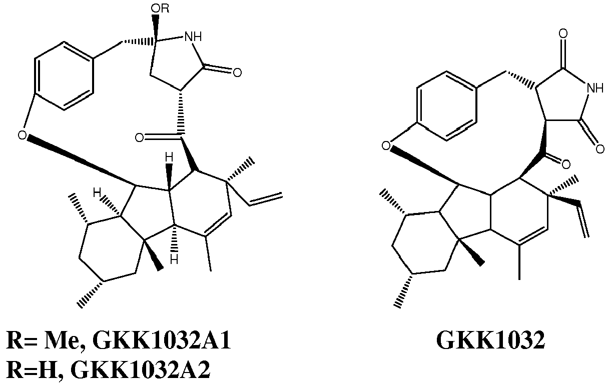

### SUMMARY OF THE INVENTION

The present invention is directed to the use of compounds of general formula: 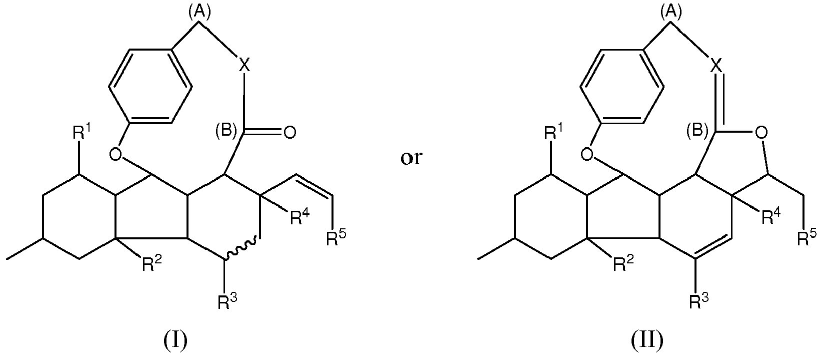 wherein
the wavy bond ∼ is either a double bond or the epoxide, 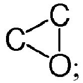
R¹, R², R³ and R⁴ are each independently selected from the group consisting of hydrogen and substituted or unsubstituted C₁-C₆ alkyl;
R⁵ is selected from the group consisting of hydrogen, substituted or unsubstituted C₁-C₆ alkyl, or halogen;
X denotes a group represented by formulas (III) to (XII), 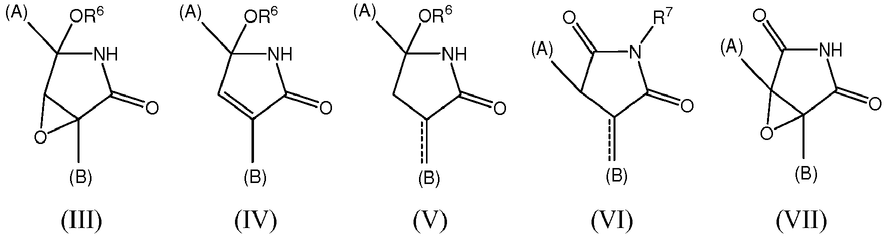 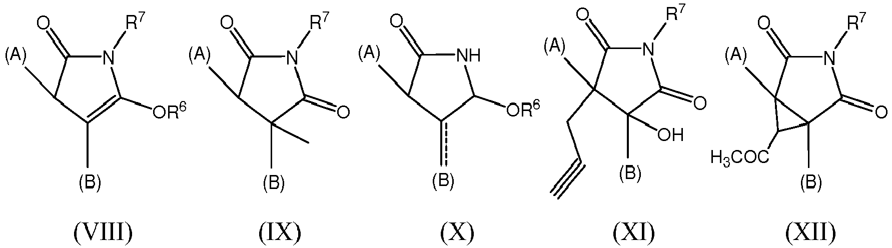
wherein
the dashed bond represents an optional double bond which, when present, is the double bond between X and (B) in the compound of formula (II);
R⁶ is selected from the group consisting of hydrogen, substituted or unsubstituted acyl, substituted or unsubstituted alkyl, substituted or unsubstituted alkenyl, substituted or unsubstituted alkynyl, substituted or unsubstituted aryl, substituted or unsubstituted aralkyl, -CONH₂, alkali metal, and sugar; and
R⁷ is selected from the group consisting of hydrogen, substituted or unsubstituted C₁-C₆ alkyl, substituted or unsubstituted C₁-C₆ alkynyl, nitrile, and -CH₂-CO-CH₃;
or pharmaceutically acceptable salts, solvates, tautomers, or stereoisomers thereof, for use in the treatment of diseases associated with oxidative stress.

In another aspect, the present invention is also directed to compounds of general formula (I) or (II), pharmaceutically acceptable salts, solvates, tautomers, stereoisomers, or mixtures thereof for use as a medicament for the treatment of diseases associated with oxidative stress.

In another aspect, the present invention is also directed to compounds of general formula (Ia) or (IIa) or pharmaceutically acceptable salts, solvates, tautomers, or stereoisomers thereof: 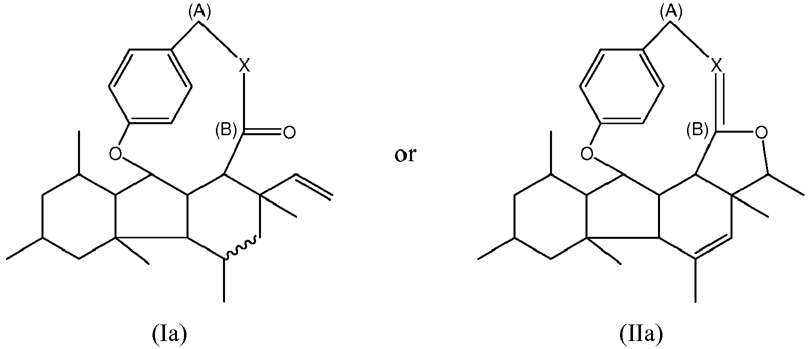 wherein
the wavy bond ∼ is either a double bond or the epoxide, 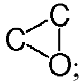 X denotes a group represented by formulas, 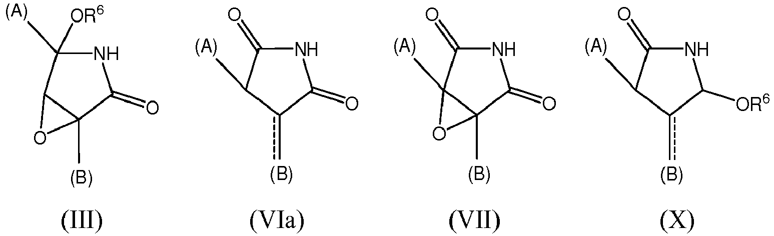 wherein
the dashed bond represents an optional double bond which, when present, is the double bond between X and (B) in the compound of formula (II);
wherein R⁶ is selected from the group consisting of hydrogen, substituted or unsubstituted acyl, substituted or unsubstituted alkyl, substituted or unsubstituted alkenyl, substituted or unsubstituted alkynyl, substituted or unsubstituted aryl, substituted or unsubstituted aralkyl, -CONH₂, alkali metal, and sugar, or a pharmaceutically acceptable salt, tautomer, solvate or stereoisomer thereof,
with the proviso that compound GKK1032, of formula 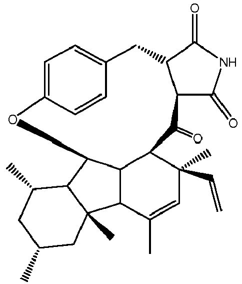 is excluded.

In another aspect, the present invention is also directed to compounds of general formula (Ia) or (IIa), pharmaceutically acceptable salts, solvates, tautomers, stereoisomers, or mixtures thereof for use as a medicament.

In another aspect, the present invention is also directed to the use of compounds of general formula (Ia) or (IIa), pharmaceutically acceptable salts, solvates, tautomers, stereoisomers or mixtures thereof in the preparation of a medicament.

Some of the diseases that can be treated with the compounds of the present invention are, in a non-limiting manner: neurodegenerative diseases such as Parkinson's disease, Alzheimer's disease, senile dementia, Huntington's disease, multiple sclerosis or amyotrophic lateral sclerosis; stroke; mitochondriopathies with neuromuscular affection; cardiovascular diseases; pancreatitis; and cirrhosis.

Other aspects of the invention are methods of treatment, and compounds for use in these methods.

Therefore, the present invention further provides a method of treating diseases associated with oxidative stress which comprises administering to a patient in need of such treatment a therapeutically effective amount of a compound of general formula (I) or (II) as defined above, or a pharmaceutically acceptable salts, solvates, tautomers, or stereoisomer thereof. Preferably, the patient in need of such treatment may be any mammal, notably a human.

The present invention also relates to the obtention of the compounds of general formula (Ia) or (IIa) from a strain of a microorganism capable of producing them.

The preferred process comprises the steps of cultivating a strain of a microorganism capable of producing compounds of general formula (Ia) or (IIa) in an aqueous nutrient medium with assimilable carbon and nitrogen sources and salts, under controlled submerged aerobic conditions, and then recovering and purifying the natural compounds according to the invention from the cultured broth or by treating the cultured broth using standard organic synthetic reactions known by the skilled person, to obtain the corresponding derivatives and then recovering and purifying these derivatives from the crude of the reaction.

The present invention also relates to the obtention of compounds of general formula (Ia) or (IIa) by synthesis from the natural compounds isolated from a strain of a microorganism capable of producing them.

The compounds of general formula (Ia) or (IIa) may be synthesised from commercially available starting materials using conventional procedures. For example, using standard organic synthetic reactions known by the skilled person.

In another aspect, the present invention is directed to pharmaceutical compositions containing a compound of general formula (Ia) or (IIa), pharmaceutically acceptable salts, or stereoisomers thereof together with a pharmaceutically acceptable carrier.

These and further aspects of the invention are defined in the claims.

### BRIEF DESCRIPTION OF DRAWINGS

**Figure 1** represents H₂O₂ formation in four different neuron groups, A) represents the untreated group, B) represents neurons treated with L-buthionine sulfoximine and rotenone, C) represents neurons treated with L-buthionine sulfoximine and rotenone and Compound I at 1 µg/ml, D) represents neurons treated with L-buthionine sulfoximine and rotenone and Compound I at 5 µg/ml.
**Figure 2** represents H₂O₂ formation in five different neuron groups, all of them incubated for 6 hours. A) represents the untreated group, B) represents neurons treated with L-buthionine sulfoximine and rotenone, C) represents neurons treated with L-buthionine sulfoximine and rotenone and Compound I at 1 µg/ml, D) represents neurons treated with L-buthionine sulfoximine and rotenone and GKK1032 at 1 µg/ml, E) represents neurons treated with L-buthionine sulfoximine and rotenone and Pyrrocidine A at 1 µg/ml.
**Figure 3** represents H₂O₂ formation in two different neuron groups, A) represents the control group, A1 untreated with Compound I and A2 pre-treated with Compound I at 5 µg/ml, B) represents neurons treated with L-buthionine sulfoximine and rotenone, B1 untreated with Compound I and B2 pre-treated with Compound I at 5 µg/ml.

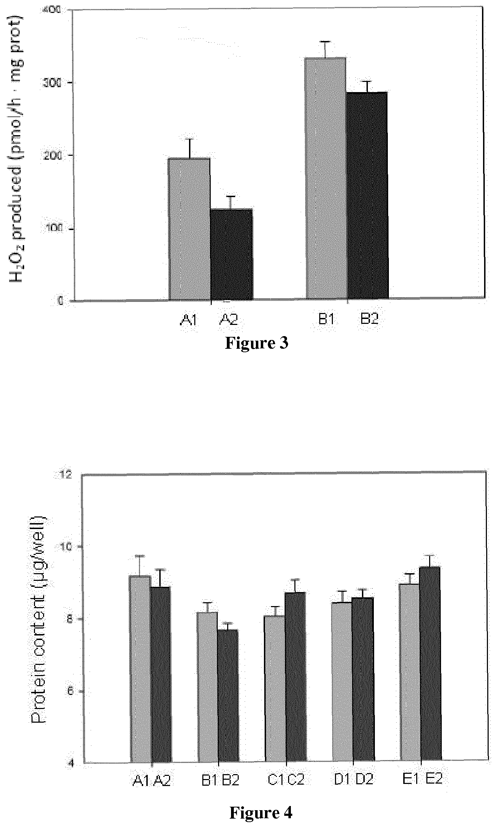

**Figure 4** represents protein content in five different neuron groups, A) represents the untreated group, A1 incubated for 3h and A2 incubated for 6h, B) represents neurons treated with L-buthionine sulfoximine and rotenone, B1 incubated for 3h and B2 incubated for 6h, C) represents neurons treated with L-buthionine sulfoximine and rotenone and Compound I at 0.1 µg/ml, C1 incubated for 3h and C2 incubated for 6h, D) represents neurons treated with L-buthionine sulfoximine and rotenone and Compound I at 1 µg/ml, D1 incubated for 3h and D2 incubated for 6h, E) represents neurons treated with L-buthionine sulfoximine and rotenone and Compound I at 5 µg/ml, E1 incubated for 3h and E2 incubated for 6h.
**Figure 5** shows the H₂O₂ production in neurons (C57BL6/J) incubated in DMSO only (DMSO empty bar), DMSO and NMDA (DMSO filled bar) and with increasing quantities of a) compound I; b) GKK1032 and c) Pyrrocidine B. *: p-value < 0.05 vs. β-amyloid + DMSO (student's test)
**Figure 6** shows the concentration of active caspase-3 in neuron (C57BL6/J) cells incubated in DMSO only (DMSO empty bar), DMSO and NMDA (DMSO filled bar) and with increasing quantities of a) compound I; b) GKK1032 and c) Pyrrocidine B. *: p-value < 0.05 vs. β-amyloid + DMSO (student's test)
**Figure 7** shows the concentration of active caspase-3 in neuron (C57BL6/J) cells incubated in DMSO only (DMSO empty bar), DMSO with β-amyloid (10 µM, 24 h) (DMSO filled bar) and with increasing quantities of compound I, compounds CL0661, CL0665, CL0666 and CL0670 (concentrations of 0.1 µM, 1 µM, 5 µM and 10 µM). *: p-value < 0.05 vs. β-amyloid + DMSO (student's test)
**Figure 8** represents the concentration of Compound I in brain of female C57BL/6 mice collected at t = 0 min, 5 min, 15 min, 30 min, 1 hour, 4 hours, 10 hours, 24 hours and 48 hours.
**Figure 9** represents the results from the rotarod test to 8 weeks-old C57BL6/J male mice separated in two groups: Control (mice injected with vehicle) and Compound I (mice injected with a once-daily dose of 150 mg/kg of Compound I).
**Figure 10** represents the open field test results of 8 weeks-old C57BL6/J male mice injected with vehicle only and mice injected with Compound I (once-daily dose of 150 mg/kg). *: p-value < 0.05 vs. control (student's test)
**Figure 11** shows the same results of the open field test results as in Figure 10 but represented in terms of the animal's activity. The top four images show the activity of the mice whereas the bottom four images represent heat maps.
**Figure 12** shows the Novel Object Recognition test of 8 weeks-old C57BL6/J male mice injected with vehicle only and mice injected with Compound I (once-daily dose of 150 mg/kg). The top image shows the preference index whereas the bottom image represents the activity maps.
**Figure 13** shows the rotarod test results for groups I (□), II (■), III (●) and IV (○).* or #: p-value < 0.05 vs. 3-nitropropionic acid (*student's test; #ANOVA, DMS post-hoc). Compound I injections were performed at day 1 and day 2. 3-nitropropionic acid injections were performed at days 1, 1.5, 2, 2.5, 3 and 3.5. The shown data points were obtained 2 hours after the injections.
**Figure 14** shows the rotarod test results in assessment of the neuroprotective effect of Compound I in mice submitted, or not, to middle cerebral artery occlusion. I - mice submitted to surgery without occlusion; II - mice submitted to surgery without occlusion and administration of Compound I; III - mice submitted to Middle Cerebral Artery occlusion and IV - mice submitted to Middle Cerebral Artery occlusion and administration of Compound I.
**Figure 15** shows the strokes sizes for the mice submitted to the test explained in Example 10. I - mice submitted to surgery without occlusion; II - mice submitted to surgery without occlusion and administration of Compound I; III - mice submitted to Middle Cerebral Artery occlusion and IV - mice submitted to Middle Cerebral Artery occlusion and administration of Compound I. The percentage values are the volume of the cerebral stroke.
**Figure 16** is the graphical representation of the volume of the cerebral stroke of mice submitted to Middle Cerebral Artery occlusion effect (empty bar) and of mice submitted to Middle Cerebral Artery occlusion and administration of Compound I (solid bar). *: p-value < 0.05 vs. ischemia (student's test)
**Figure 17** shows the ¹H-NMR spectrum (CDCl₃) of compound CL0661
**Figure 18** shows the ¹³C-NMR spectrum (CDCl₃) of compound CL0661
**Figure 19** shows the ¹H-NMR spectrum (CDCl₃) of compound CL0665
**Figure 20** shows the ¹³C-NMR spectrum (CDCl₃) of compound CL0665
**Figure 21** shows the ¹H-NMR spectrum (CDCl₃) of compound CL0666
**Figure 22** shows the ¹³C-NMR spectrum (CDCl₃) of compound CL0666
**Figure 23** shows the ¹H-NMR spectrum (CDCl₃) of compound CL0670
**Figure 24** shows the ¹³C-NMR spectrum (CDCl₃) of compound CL0670

### DETAILED DESCRIPTION OF THE INVENTION

The present invention is directed to a compound of general formula 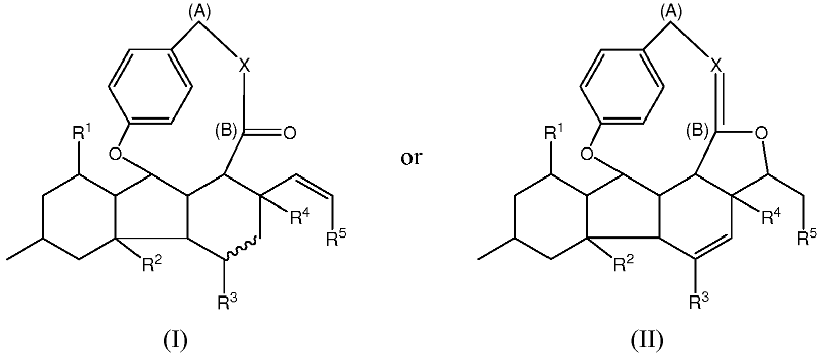 wherein
the wavy bond ∼ is either a double bond or the epoxide, 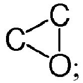
R¹, R², R³ and R⁴ are each independently selected from the group consisting of hydrogen and substituted or unsubstituted C₁-C₆ alkyl;
R⁵ is selected from the group consisting of hydrogen, substituted or unsubstituted C₁-C₆ alkyl, or halogen;
X denotes a group represented by formulas (III) to (XII), 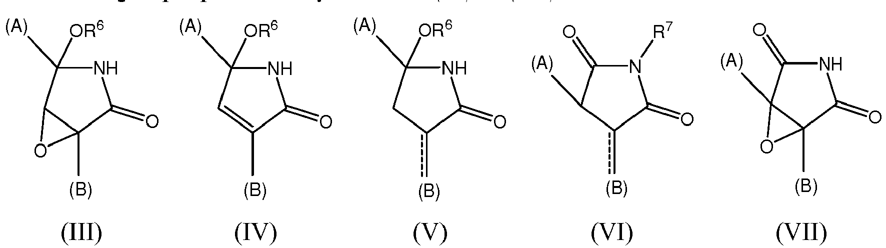 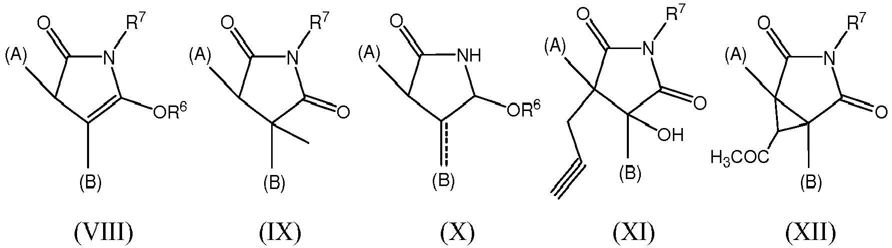
wherein
the dashed bond represents an optional double bond which, when present, is the double bond between X and (B) in the compound of formula (II);
R⁶ is selected from the group consisting of hydrogen, substituted or unsubstituted acyl, substituted or unsubstituted alkyl, substituted or unsubstituted alkenyl, substituted or unsubstituted alkynyl, substituted or unsubstituted aryl, substituted or unsubstituted aralkyl, -CONH₂, alkali metal, and sugar; and
R⁷ is selected from the group consisting of hydrogen, substituted or unsubstituted C₁-C₆ alkyl, substituted or unsubstituted C₁-C₆ alkynyl, nitrile, and -CH₂-CO-CH₃;
or pharmaceutically acceptable salts, solvates, tautomers, or stereoisomers thereof, for use in the treatment of diseases associated with oxidative stress.

The present invention is also directed to the use of a compound of general formula (I) or (II) as defined above, or pharmaceutically acceptable salts, solvates, tautomers, or stereoisomers thereof, in the preparation of a medicament for the treatment of diseases associated with oxidative stress.

The present invention is also directed to a method for the treatment of diseases associated with oxidative stress wherein the method comprises the administration of a therapeutically effective amount of a compound of general formula (I) or (II) as defined above, or pharmaceutically acceptable salts, solvates, tautomers, or stereoisomers thereof, to a person in need thereof.

The terms (A) and (B) in formulae (III)-(XII) refer to the position through which group X is bonded to the rest of the molecule.

In another aspect, the present invention is directed to compounds of general formula (Ia) or (IIa) or pharmaceutically acceptable salts, solvates, tautomers, or stereoisomers thereof: 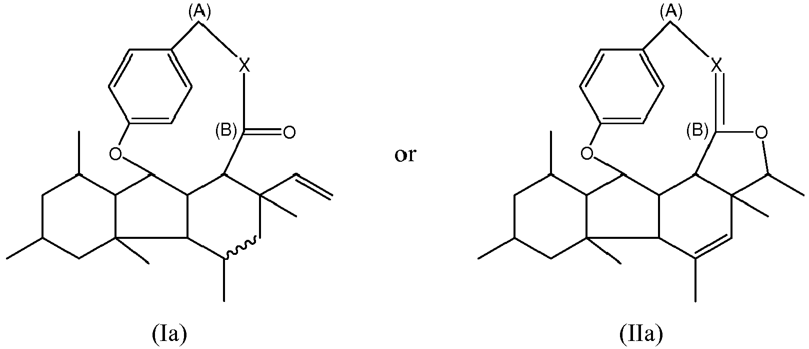 wherein
the wavy bond ∼ is either a double bond or the epoxide, 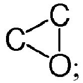 X denotes a group represented by formulas, 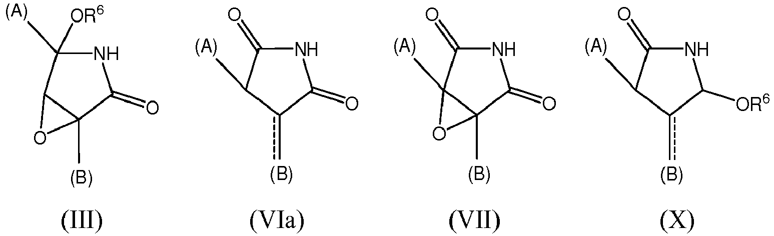 wherein
the dashed bond represents an optional double bond which, when present, is the double bond between X and (B) in the compound of formula (II);
wherein R⁶ is selected from the group consisting of hydrogen, substituted or unsubstituted acyl, substituted or unsubstituted alkyl, substituted or unsubstituted alkenyl, substituted or unsubstituted alkynyl, substituted or unsubstituted aryl, substituted or unsubstituted aralkyl, -CONH₂, alkali metal, and sugar, or a pharmaceutically acceptable salt, tautomer, solvate or stereoisomer thereof,
with the proviso that compound GKK1032, of formula 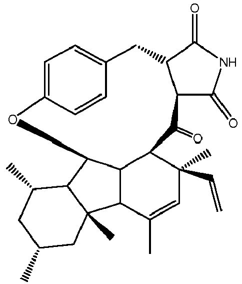 is excluded.

In the present description, the following terms have the meaning indicated:
The epoxide, 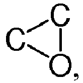 is to be understood as the three-atom cyclic ether ring which is connected to the 6-member ring of the compounds of formula (I) by each of the two carbon atoms of the epoxide.

Alkyl groups preferably have from 1 to about 20 carbon atoms, more preferably from 1 to about 12 carbon atoms, even more preferably from 1 to about 6. Alkyl groups having 1, 2, 3, 4 or 5 carbon atoms are particularly preferred. Methyl, ethyl, n-propyl, isopropyl and butyl, including n-butyl, tert-butyl, sec-butyl and iso-butyl are particularly preferred alkyl groups in the compounds of the present invention. As used herein, the term alkyl, unless otherwise indicated, refers to both cyclic and non-cyclic groups, although cyclic groups will comprise at least three carbon ring members. Non-cyclic alkyl refers to a straight-chain or branched alkyl group.

Preferred alkenyl and alkynyl groups in the compounds of the present invention have one or more unsaturated linkages and from 2 to about 20 carbon atoms, more preferably from 2 to about 12 carbon atoms, even more preferably from 2 to about 6. The terms alkenyl and alkynyl as used herein refer to both cyclic and non cyclic groups, although cyclic groups will comprise at least three or five carbon ring members, respectively. Non-cyclic alkenyl or alkynyl refers to a straight-chain or branched alkenyl or alkynyl groups. A preferred non-cyclic alkynyl group of the invention is -CH-CC.

Aryl refers to single and multiple aromatic ring radicals, including multiple ring radicals containing separate and/or fused aryl groups. Typical aryl groups contain from 1 to 3 separated or fused rings and from 6 to about 18 carbon ring atoms, such as phenyl, naphthyl, indenyl, phenanthryl or anthracyl radicals. The aryl groups in the compounds of the present invention may be substituted at one or more available positions by one or more suitable groups.

An acyl group is of the form R⁸CO-, wherein R⁸ is an organic group such as an alkyl or an aryl group as defined before e.g. acetyl, propionyl, benzoyl and the like. Suitable acyl groups have from 2 to about 12 carbon atoms, preferably from 2 to about 8 carbon atoms, more preferably from 2 to about 6 carbon atoms, most preferably 2 carbon atoms.

Notable alkali-metals include sodium or potassium.

Aralkyl refers to an aryl group linked to an alkyl radical as defined above. A preferred aralkyl group is benzyl.

Halogen refers to F, Cl, Br, I. A preferred halogen is Br.

Sugar refers to mono-, di- or tri- saccharides or saccharide derivatives, preferably mono- or di- saccharides. Pentose or hexose compounds are preferred. Derivatives include sugar glycosides, N-glycosylamines, O-acyl derivatives, O-methyl derivatives, sugar alcohols, sugar acids, and deoxy sugars.

A nitrile is an organic compound that has a -C≡N functional group, preferably within the context of the present invention, a nitrile is a -CH₂-C≡N group.

The groups above mentioned may be substituted at one or more available positions by one or more suitable groups such as OR', =O, SR', SOR', SO₂R', OSO₂R', OSO₃R', NO₂, NHR', N(R')₂, =N-R', N(R')COR', N(COR')₂, N(R')SO₂R', N(R')C(=NR')N(R')R', N₃, CN, halogen, COR', COOR', OCOR', OCOOR', OCONHR', OCON(R')₂, CONHR', CON(R')₂, CON(R')OR', CON(R')SO₂R', PO(OR')₂, PO(OR')R', PO(OR')(N(R')R'), substituted or unsubstituted C₁-C₁₂ alkyl, substituted or unsubstituted C₂-C₁₂ alkenyl, substituted or unsubstituted C₂-C₁₂ alkynyl, substituted or unsubstituted aryl, and substituted or unsubstituted aralkyl, wherein each of the R' groups is independently selected from the group consisting of hydrogen, OH, O-Sugar, NO₂, NH₂, SH, CN, halogen, =O, COH, COalkyl, COOH, CO₂Alkali-metal, substituted or unsubstituted C₁-C₁₂ alkyl, substituted or unsubstituted C₂-C₁₂ alkenyl, substituted or unsubstituted C₂-C₁₂ alkynyl and substituted or unsubstituted aryl. Where such groups are themselves substituted, the substituents may be chosen from the foregoing list.

The term "pharmaceutically acceptable salts", refers to any salt which, upon administration to the patient is capable of providing (directly or indirectly) a compound as described herein. It will be appreciated that non-pharmaceutically acceptable salts also fall within the scope of the invention since those may be useful in the preparation of pharmaceutically acceptable salts. The preparation of salts and derivatives can be carried out by methods known in the art.

For instance, pharmaceutically acceptable salts of compounds provided herein can be synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the same. Generally, nonaqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include inorganic salts such as, for example, sodium, potassium, calcium and ammonium salts, and organic alkali salts such as, for example, ethylenediamine, ethanolamine, N,N-dialkylenethanolamine, triethanolamine and basic aminoacids salts.

The compounds of the invention may be in crystalline form either as free compounds or as solvates (e.g. hydrates, alcoholates, particularly methanolates) and it is intended that both forms are within the scope of the present invention. Methods of solvation are generally known within the art. The compounds of the invention may present different polymorphic forms, and it is intended that the invention encompasses all such forms.

Any compound that is a derivative of a compound of general formula (I) or (II) is within the scope and spirit of the invention. The term "derivative" is used in its broadest sense and encompasses those compounds that are converted in vivo to the compounds of the invention. Examples of derivatives include, but are not limited to, derivatives and metabolites of the compounds of general formula (I) or (II) that include biohydrolyzable moieties such as biohydrolyzable amides, biohydrolyzable esters, biohydrolyzable carbamates, biohydrolyzable carbonates, biohydrolyzable ureides, and biohydrolyzable phosphate analogues. Preferably, derivatives of compounds with carboxyl functional groups are the lower alkyl esters of the carboxylic acid. The carboxylate esters are conveniently formed by esterifying any of the carboxylic acid moieties present on the molecule. Derivatives can typically be prepared using well-known methods, such as those described by Burger "Medicinal Chemistry and Drug Discovery 6th ed. (Donald J. Abraham ed., 2001, Wiley) and "Design and Applications of Prodrugs" (H. Bundgaard ed., 1985, Harwood Academic Publishers).

Any compound referred to herein is intended to represent such specific compound as well as certain variations or forms. The term "stereoisomer" as used herein includes any enantiomer, diastereomer or geometric isomer (E/Z) of such compound. In particular, compounds referred to herein may have asymmetric centres and therefore exist in different enantiomeric or diastereomeric forms. Thus any given compound referred to herein is intended to represent any one of a racemate, one or more enantiomeric forms, one or more diastereomeric forms, and mixtures thereof. Likewise, stereoisomerism or geometric isomerism about the double bond is also possible, therefore in some cases the molecule could exist as (E)-isomer or (Z)-isomer (trans and cis isomers). If the molecule contains several double bonds, each double bond will have its own stereoisomerism, that could be the same or different than the stereoisomerism of the other double bonds of the molecule. All the stereoisomers including enantiomers, diastereoisomers and geometric isomers of the compounds referred to herein, and mixtures thereof, are considered within the scope of the present invention.

Furthermore, any compound referred to herein may exist as tautomers. Specifically, the term tautomer refers to one of two or more structural isomers of a compound that exist in equilibrium and are readily converted from one isomeric form to another. Common tautomeric pairs are amine-imine, amide-imidic acid, keto-enol, lactam-lactim, etc.

Additionally, any compound referred to herein is intended to represent hydrates, solvates, and polymorphs, and mixtures thereof when such forms exist in the medium. All geometric isomers, tautomers, atropisomers, hydrates, solvates, polymorphs, and isotopically labelled forms of the compounds referred to herein, and mixtures thereof, are considered within the scope of the present invention.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon or ¹⁵N-enriched nitrogen are within the scope of this invention.

An important feature of the above described compounds of formula (I) or (II) is their bioactivity and in particular their antioxidant and neuroprotective properties.

Therefore, the present invention provides compounds and pharmaceutical compositions that are useful for the treatment of diseases or conditions associated with oxidative stress. Among such diseases or conditions are included neurodegenerative diseases such as Parkinson's disease, Alzheimer's disease, senile dementia, Huntington's disease, multiple sclerosis or amyotrophic lateral sclerosis, stroke, mitochondriopathies with neuromuscular affection, cardiovascular disease, pancreatitis, and cirrhosis.

Pharmaceutical compositions useful for the method of the invention comprise a compound of formula (I), (II), or mixtures thereof, a pharmaceutically acceptable salt, solvate, tautomer, or stereoisomer thereof together with a pharmaceutically acceptable carrier for administration to a patient.

The term "carrier" refers to a diluent, adjuvant, excipient or vehicle with which the active ingredient is administered. Suitable pharmaceutical carriers are described in "Remington's Pharmaceutical Sciences" by E. W. Martin, 1995.

Examples of pharmaceutical compositions include any solid (tablets, pills, capsules, granules etc.) or liquid (solutions, suspensions or emulsions) composition for oral, topical or parenteral administration.

In a preferred embodiment the pharmaceutical compositions are in oral form, either solid or liquid. Suitable dose forms for oral administration may be tablets, capsules, syrups or solutions and may contain conventional excipients known in the art such as binding agents, for example syrup, acacia, gelatine, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycolate or microcrystalline cellulose; or pharmaceutically acceptable wetting agents such as sodium lauryl sulfate.

The solid oral compositions may be prepared by conventional methods of blending, filling or tableting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the appropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

Administration of the compounds of formula (I), (II) or compositions thereof may be by any suitable method, such as intravenous infusion, oral preparations, and intraperitoneal and intravenous administration. Oral administration is preferred because of the convenience for the patient and the chronic character of the diseases to be treated.

Generally an effective administered amount of a compound of formula (I) or (II) will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day.

The compounds of general formula (I) or (II) and compositions thereof may be used with other drug(s) to provide a combination therapy. The other drug(s) may form part of the same composition, or be provided as a separate composition for administration at the same time or at different times.

In a particular embodiment of the invention, R¹, R², R³, R⁴ and R⁵ are each independently selected from the group consisting of hydrogen and substituted or unsubstituted C₁-C₃ alkyl. Preferably, R¹, R², R³, R⁴ and R⁵ are each independently selected from the group consisting of hydrogen and methyl.

In an embodiment, at least one, preferably at least two, more preferably at least three of R¹, R², R³, R⁴ and R⁵ are independently selected from substituted or unsubstituted C₁-C₆ alkyl. In a particular embodiment, said substituted or unsubstituted C₁-C₆ alkyl group is a substituted or unsubstituted C₁-C₃ alkyl. Preferably it is methyl.

In another embodiment, R¹, R², R³, R⁴ and R⁵ are independently selected from substituted or unsubstituted C₁-C₆ alkyl, preferably from substituted or unsubstituted Ci-C₃ alkyl, more preferably they are methyl.

In a particular embodiment of the invention, R⁶ is selected from the group consisting of hydrogen, substituted or unsubstituted C₂-C₁₂ acyl, substituted or unsubstituted C₁-C₆ alkyl, substituted or unsubstituted C₂-C₆ alkenyl, substituted or unsubstituted C₂-C₆ alkynyl, substituted or unsubstituted C₆-C₁₀ aryl, substituted or unsubstituted (C₆-C₁₀)aryl(C₁-C₆)alkyl, -CONH₂, alkali metal, and sugar.

In another embodiment, R⁶ is selected from the group consisting of hydrogen, substituted or unsubstituted -C(O)(C₁-C₆)alkyl, substituted or unsubstituted C₁-C₃ alkyl, substituted or unsubstituted C₂-C₄ alkenyl, substituted or unsubstituted C₂-C₄ alkynyl, substituted or unsubstituted C₆-C₁₀ aryl and substituted or unsubstituted (C₆-C₁₀)aryl(C₁-C₃)alkyl.

In a further embodiment, R⁶ is selected from the group consisting of hydrogen, substituted or unsubstituted acetyl, substituted or unsubstituted methyl, ethyl, phenyl and substituted or unsubstituted benzyl.

According to an embodiment, R⁶ is selected from the group consisting of hydrogen, acetyl, methyl, ethyl, phenyl and benzyl. In a preferred embodiment, R⁵ is hydrogen.

In a particular embodiment of the invention, X represents a group represented by formulas, 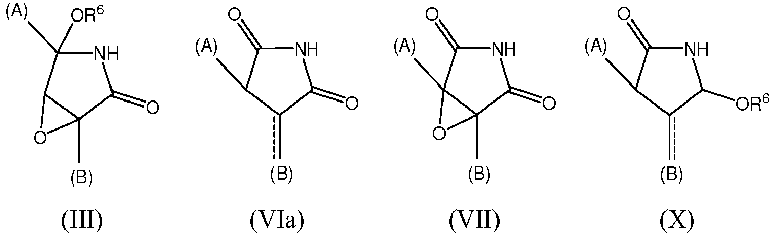 wherein R⁶ and the dashed bond are as defined previously.

According to an embodiment of the first aspect of the invention, the compound of formula (I) or (II) for use is selected from: 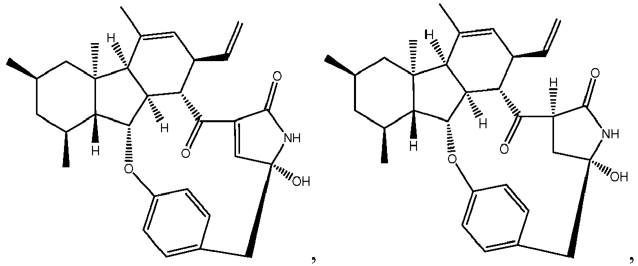 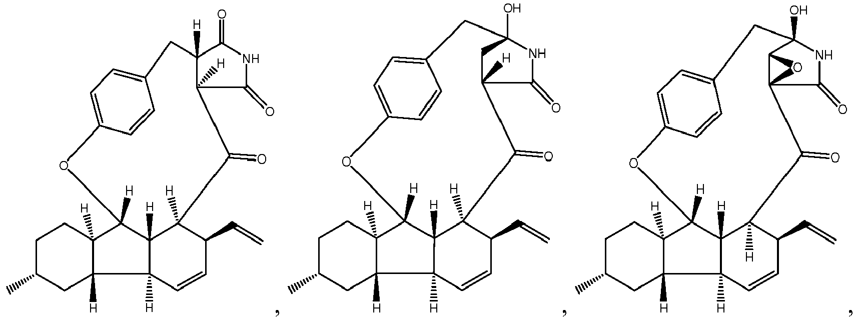 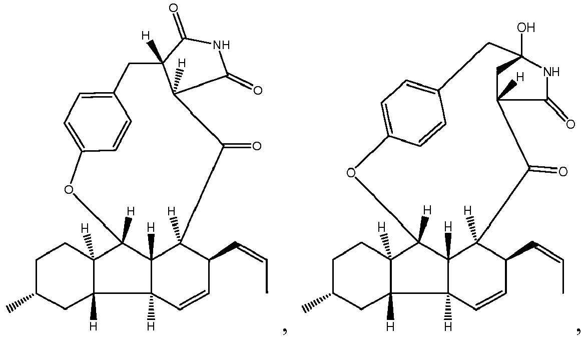 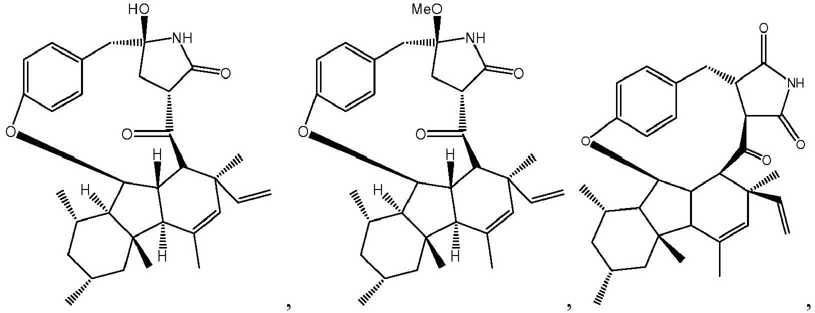 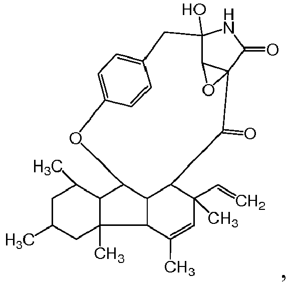 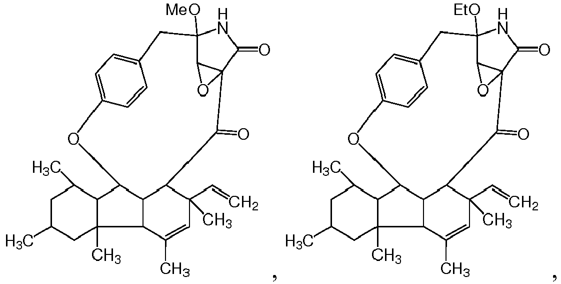 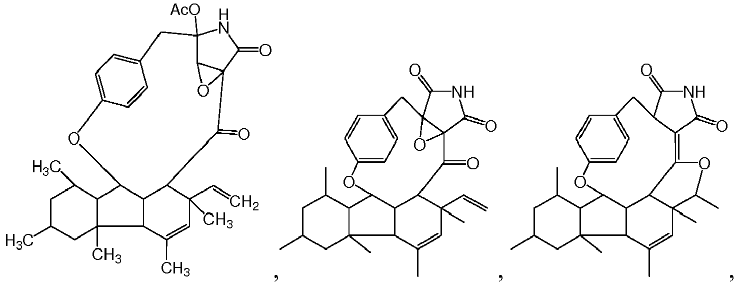 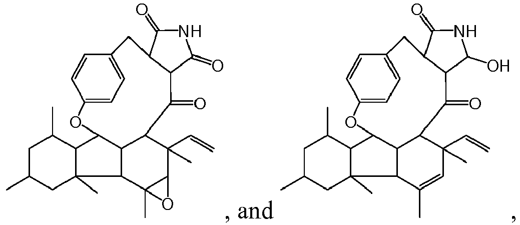 or pharmaceutically acceptable salts, solvates, tautomers, or stereoisomers thereof.

Preferably, the compound of formula (I) or (II) for use is selected from: 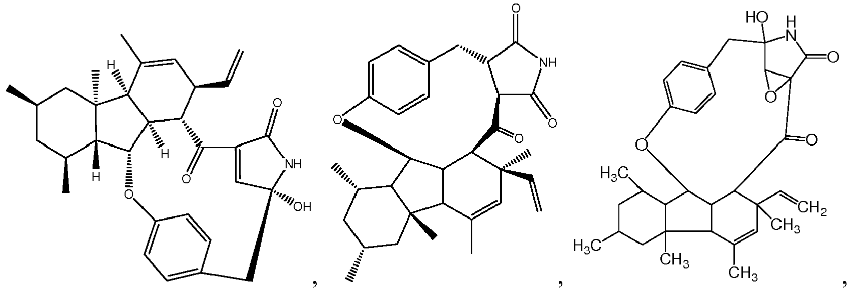 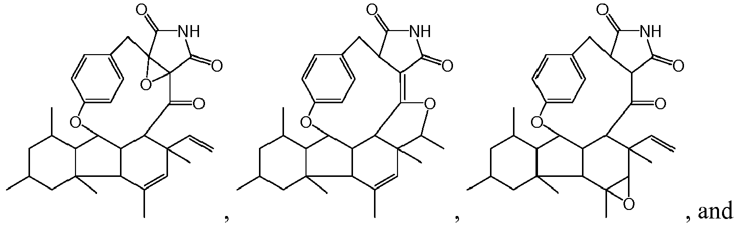 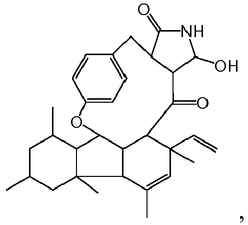 or pharmaceutically acceptable salts, solvates, tautomers, or stereoisomers thereof. In one embodiment, the compounds of the invention are preferably those in which the general formula is (Ia) or (IIa) 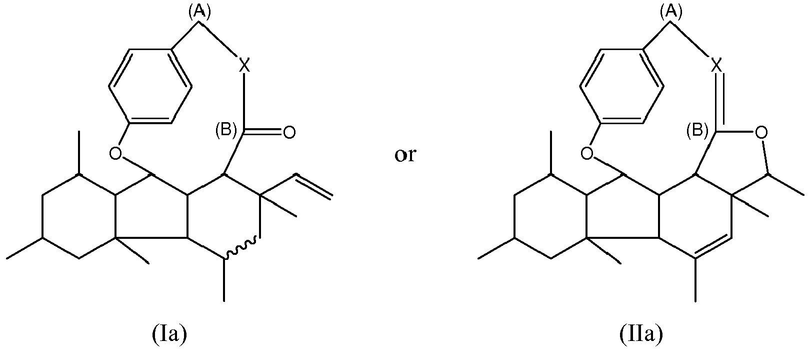 wherein X denotes a group represented by formulas, 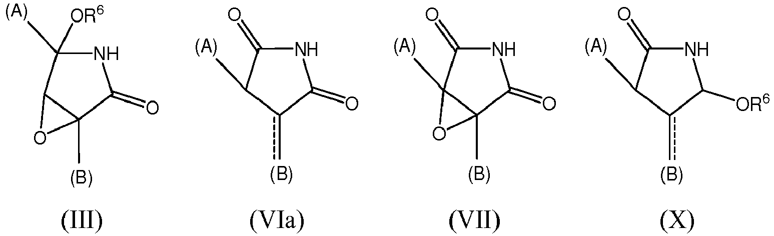 and wherein R⁶, the wavy bond and the dashed bond are as defined previously, or pharmaceutically acceptable salts, solvates, tautomers, or stereoisomers thereof.

In a particular embodiment, the compound of formula (Ia) or (IIa) is selected from: 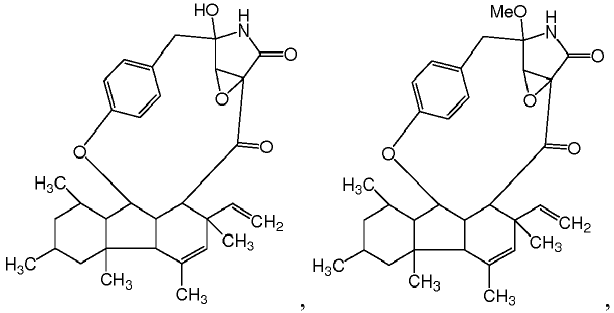 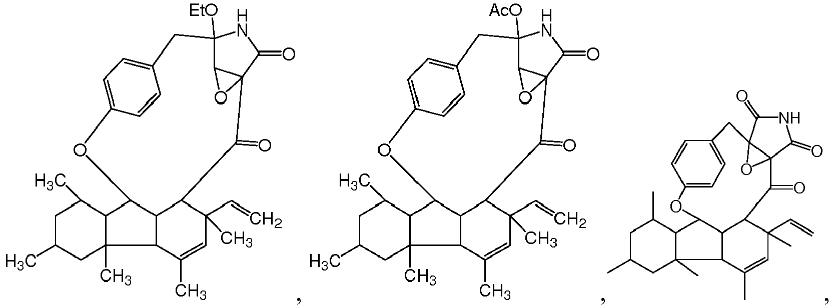 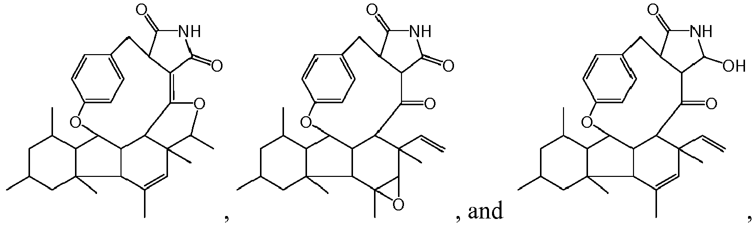 or pharmaceutically acceptable salts, solvates, tautomers, or stereoisomers thereof.

Particularly preferred compounds of the invention falling under the general formula (Ia) or (IIa) are Compounds: 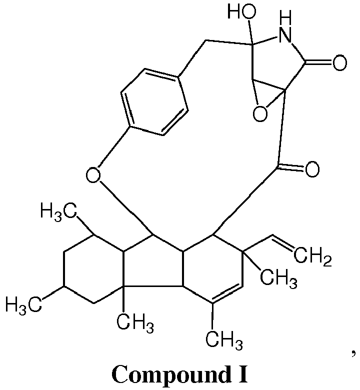 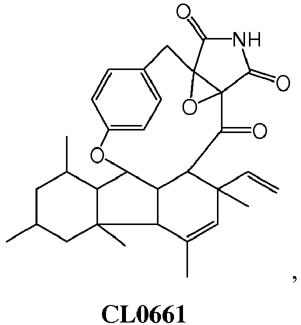 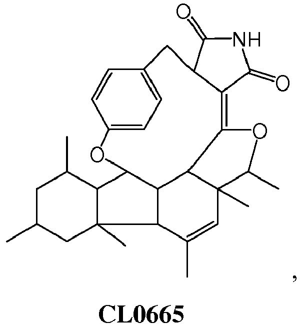 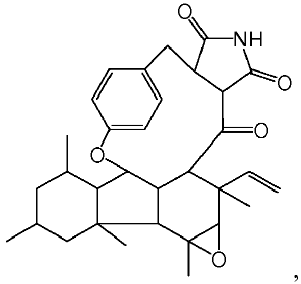 and

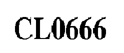

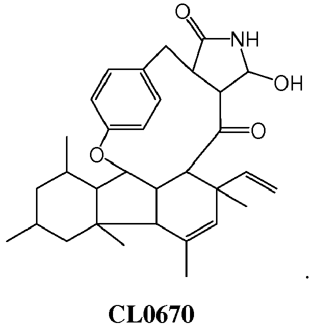

Compounds of formula (Ia) or (IIa) can be produced using microorganisms. Preferably, the organism used for the production of compounds of formula (Ia) or (IIa) is the fungal strain HT-09B-VENT-WQ002, a culture of which has been deposited in the Colección Española de Cultivos Tipo at the Universidad de Valencia, Spain under the accession number CECT 20769. This deposit has been made under the provisions of the Budapest Treaty and all restrictions on the availability thereof to the public will be irrevocably maintained upon the granting of a patent on this application.

The organism was isolated from a soil sample collected at El Ventorrillo, Madrid, Spain.

While the deposited strain is clearly preferred, the present invention is not restricted or limited to any particular strain or organisms. It is the intention of the present invention to include other producing organisms of compounds of formula (Ia) or (IIa), strains or mutants within the scope of this invention.

HT-09B-VENT-WQ002 cultured under controlled conditions in a suitable medium produces the Compound (I). This strain is preferably grown in an aqueous nutrient medium, under aerobic and mesophilic conditions. The optimal temperature for growing it on solid media is 24-28°C. The pH range for growing is from 5 to 7. Growth was best with glucose and starch. Other carbon sources such as flour, glycerol, and dextrose can also be used. In shaken cultures, the mycelium grows highly dense.

### Culture characteristics:

Colonies reach 4 cm diameter in ten days at 24°C on potato dextrose agar, in a culture chamber that maintains a humidity of 42%.

### Colony characteristics:

Colonies grow moderately, white, hairy to cottony. It produces a yellow pigment at submerged hyphae.

### Microscopy:

Conidiophores erect, hialine, bearing divergent whorls of metulae without branches. Conidia globose smooth-walled, 2-3 µm diameter.

Taxonomy classification was achieved after a sequencing analysis of ITS1-5.8S-ITS2 ribosomal DNA region. The sequence was confronted with the Gene Bank depository using the Blastn algorithm. Strain HT-09B-VENT-WQ002 has a 100% match when compared with *Penicillium citrinum.*

### Fermentation:

HT-09B-VENT-WQ002 when cultured under controlled conditions in a suitable medium produces compounds of general formula (Ia) or (IIa). This strain is preferably grown in an aqueous nutrient medium, under aerobic and mesophilic conditions, preferably at 24°C-28°C, and at a pH ranging between 5.0 and 7.0. A wide variety of liquid culture media can be utilized for the cultivation of the organism. Useful media are those that include an assimilable carbon source, such as starch, dextrin, glucose, and the like, an assimilable nitrogen source such as protein, protein hydrolysate, defatted meals, corn steep, and the like, and useful inorganic anions and cations such as sodium, magnesium, potassium, ammonium, sulfate, chloride, phosphate, carbonate, and the like. Trace elements may be added also. Aeration is preferably achieved by supplying air to the fermentation medium. Agitation is provided by a mechanical impeller.

The required steps needed for the production of compounds of formula (Ia) or (IIa) by the preferred organism are: start with frozen or lyophilized mycelium. Obtain mycelial mass culturing the initial cells in shake flasks with a culture medium containing some of the ingredients described above at mesophilic temperatures and in aerobic conditions. This inoculum is used to start a culture in flasks with higher volume. Sometimes the production medium may be different than the one used as inoculum.

Other compounds of this invention can be prepared using techniques already known in the art. As examples, said compounds can be made by modifications of the conditions of the fermentation or by conventional chemical methods using compounds of formula (I) or (II) as starting materials, or by synthesis, from commercially available products and reagents, using standard organic synthesis reactions known by the skilled person such as those described in "Comprehensive Organic Transformations: A Guide to Functional Group Preparations", 2nd Edition, Richard C. Larock, or in "March's Advanced Organic Chemistry: Reactions, Mechanisms, and Structure", 6th Edition , Michael B. Smith, and Jerry March, or in "Advanced Organic Chemistry, Part B: Reactions and Synthesis", 5th Edition, Francis A. Carey, and Richard J. Sundberg.

As examples of derivatives of the natural products of formula (Ia) or (IIa) obtainable by conventional chemical methods, compounds A and B could be obtained by treatment of Compound I, with MeOH/HCl and EtOH/HCl, respectively.

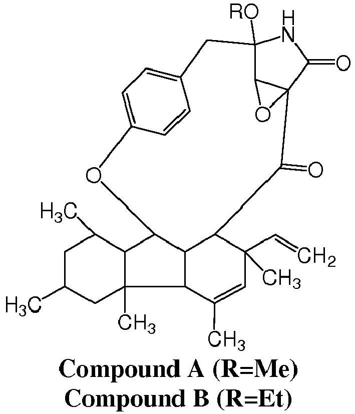

Compound C could be obtained by treatment of Compound I with Ac₂O and Pyridine.

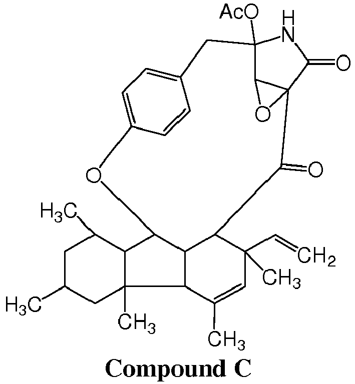

The invention will be further illustrated by means of examples. These should be interpreted merely as illustrative of the invention. The invention will be illustrated below by means of tests performed by the inventors which clearly show the usefulness of compounds of formula (I) or (II) in the applications defined by the claims.

### EXAMPLES

### Example 1: Fermentation of HT-09B-VENT-WQ002

### Stock culture:

A pure culture of HT-09B-VENT-WQ002 was kept frozen at -70°C in 20% glycerol.

### Inoculum:

A well grown agar culture was used to inoculate 40 mL of seed medium containing 2% oat meal, 2% malt extract, 0.01% KH₂PO₄, 0.005% MgSO₄ and tap water in 250 mL shake flasks, and cultured at 24°C on a rotary shaker at 200 rpm. The flasks were incubated 48 hours, and used as a first stage inoculum.

### Fermentation:

250 mL of the same medium in 2L Erlenmeyer flasks were inoculated with 10% of the first stage inoculum. The fermentation was carried out for 7 days at 24°C on a rotary shaker at 200 rpm. Production of Compound I can be monitored by HPLC or any other method with enough sensitivity.

### Example 2: Production of Compound I and GKK1032

Fermentation broth (4L) of fungus HT-09B-VENT-WQ002 was filtered through Celite and the mycelial cake extracted twice with 2L of a mixture of EtOAc/MeOH (3:1). The resultant suspension was filtered and partitioned between EtOAc and water. The organic layer was taken to dryness and the crude extract (3.1 g) was fractionated by VFC (vacuum flash chromatography) on silica gel, eluted with a stepwise gradient of Hexane/EtOAc/MeOH. Fractions 7-8 (eluted with EtOAc and EtOAc/MeOH 9:1, 0.4 g) were applied to a silica gel column and flash-chromatographied by elution with Hexane/EtOAc gradient. Fraction 11 (eluted with hexane/EtOAc 6:4, 32 mg) was finally purified by semi-preparative reversed-phase HPLC (Agilent Prep 1100 HPLC, 5-µg C18 column; 30x100 mm; eluting with a 25 min gradient of 40-100 aqueous MeOH at a flow rate of 21.6 mL/min, with UV detection at 200 nm) to give 19 mg of Compound I (Rt: 17.64 min). Fraction 6 (eluted with Hexane/EtOAc 8:2, 32 mg) was purified following the same protocol to give 9.5 mg of the known compound GKK1032 (Rt: 18.57 min). Retention times relate to analysis in an Agilent 1200 HPLC, Zorbax XDB C18, 1.8 µm column; 4.6 x 50 mm; eluting with a 20 min gradient of 15-100 aqueous MeOH, containing 0.1% trifluoroacetic acid at a flow rate of 0.5 mL/min, at 20°C, with UV detection at 220 nm.

Compound I has a molecular formula of C₃₂H₃₉NO₅ established by APCI and API-ES mass spectra ([M+H]⁺ at m/z 518), ¹³C NMR, and DEPT data. The complete assignments of ¹H and ¹³C NMR spectra of Compound I were finally established by 2D NMR experiments (COSY, HSQC and HMBC), and its spectroscopic data are shown in table 1.

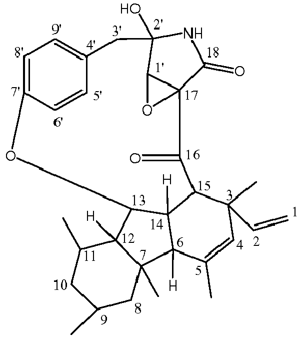

**Table 1. ¹H and ¹³C NMR Spectral Data of Compound I [δ (ppm), J_{HH} (Hz); CDCl₃] and HMBC**

| Position | ¹³C (δ) | ¹H (δ) | HMBC |
|---|---|---|---|
| 1 | 115.2 | 4.93 (1H, d, 17.3 Hz) | C2, C3 |
| | | 5.03 (1H, d, 10.8 Hz) | C3 |
| 2 | 144.3 | 5.71 (1H, dd, 17.5, 10.8 Hz) | C4, 3-CH₃ |
| 3 | 44.0 | | |
| 4 | 128.8 | 5.21 (1H, brs) | |
| 5 | 138.7 | | |
| 6 | 52.7 | 2.04 (1H, m) | |
| 7 | 41.7 | | |
| 8 | 48.8 | 0.84 (1H, t, 11.8 Hz) | |
| | | 1.96 (1H, m) | |
| 9 | 28.2 | 1.85 (1H, m) | |
| 10 | 45.8 | 0.62 (1H, q, 11.7 Hz) | |
| | | 1.82 (1H, m) | |
| 11 | 27.4 | 1.85 (1H, m) | |
| 12 | 61.1 | 1.18 (1H, m) | |
| 13 | 87.8 | 4.69 (1H, dd, 8.1, 5.6 Hz) | C7, C7' |
| 14 | 50.3 | 2.58 (1H, ddd, 12.9, 7.4, 5.6 Hz) | C6, C13, C15, C16 |
| 15 | 58.4 | 3.79 (1H, d, 7.4 Hz) | C3, C4, C13, C14, C16, 3-CH₃ |
| 16 | 201.9 | | |
| 17 | 59.0 | | |
| 18 | 168.6 | | |
| 1' | 66.3 | 3.76 (1H, d, 2.6 Hz) | C2' |
| 2' | 84.3 | | |
| 3' | 43.9 | 3.08 (1H, d, 13.3 Hz) | C1', C2', C4', C5', C9' |
| | | 3.15 (1H, d, 13.3 Hz) | C1', C2', C4', C5', C9' |
| 4' | 126.8 | | |
| 5' | 132.3 | 7.07 (1H, dd, 8.5, 2.1 Hz) | C4', C7', C9' |
| 6' | 122.7 | 6.74 (1H, dd, 8.5, 2.5 Hz) | |
| 7' | 158.7 | | |
| 8' | 118.8 | 7.14 (1H, dd, 8.5, 2.5 Hz) | C6' |
| 9' | 131.7 | 7.05 (1H, dd, 8.5, 2.1 Hz) | C4', C5', C7' |
| 3-CH₃ | 26.5 | 1.27 (3H, s) | C2, C3, C4, C15 |
| 5-CH₃ | 21.0 | 1.90 (3H, s) | C4, C5, C6 |
| 7-CH₃ | 16.3 | 1.15 (3H, s) | C6, C7, C8, C12 |
| 9-CH₃ | 23.0 | 0.91 (3H, d, 6.0 Hz) | C8, C9, C10 |
| 11-CH₃ | 19.9 | 1.06 (3H, d, 6.4 Hz) | C10, C11, C12 |
| NH | | 5.85 (1H, brs) | C17, C1' |

### Example 3: Production of Compounds of formula (I) or (II)

A fermentation broth (8L) of fungus HT-09B-VENT-WQ002 was submitted to a solid phase extraction using the adsorption resin XAD-1180 as stationary phase and EtOAc/MeOH 3:1 as mobile phase. LCMS allowed the isolation of compound I (106 mg, 96% purity), GKK1032 (3.5 mg, 70% purity), pyrrocidine A (15.8 mg, 77% purity), pyrrocidine B (5.63 mg, 94% purity) and GKK-1032 A2 (7.67 mg, 53% purity).

Five further fermentation broths were conducted (total volume 49.5 L) and 3 g of GKK1032 was isolated. This compound was submitted to different reactions, mainly with low molecular weight organometallics, acids, bases, alkylating agents and reducing agents to yield 14 compounds, structurally related to compound I:

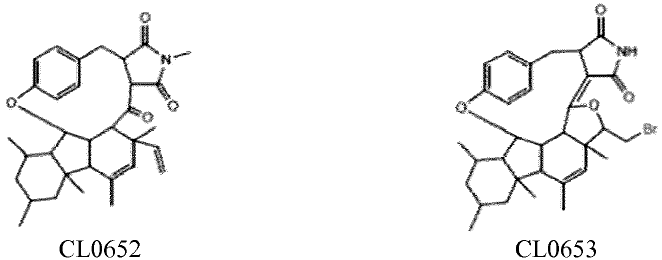

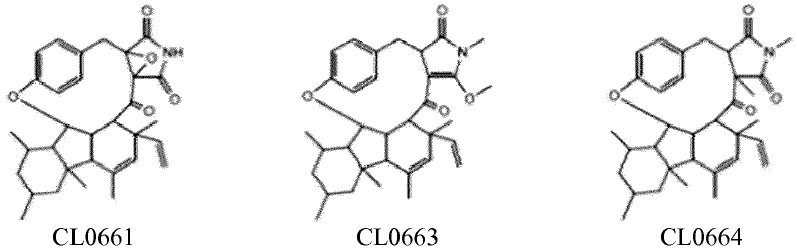

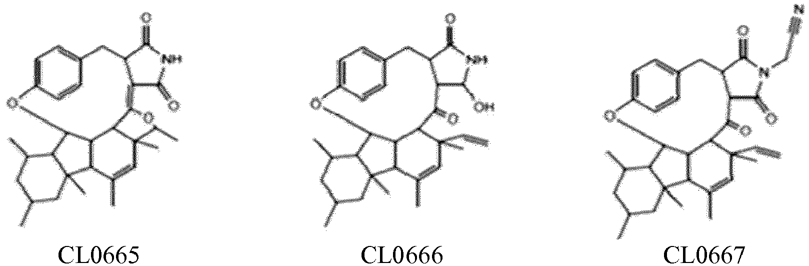

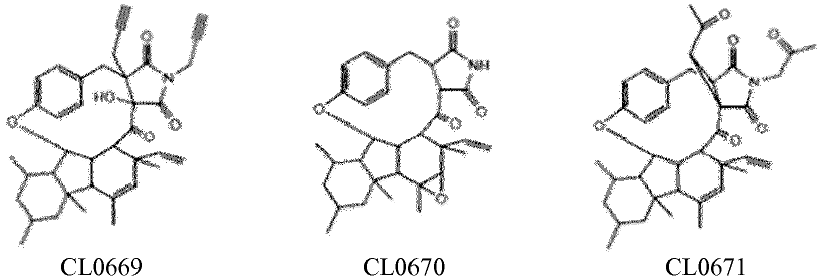

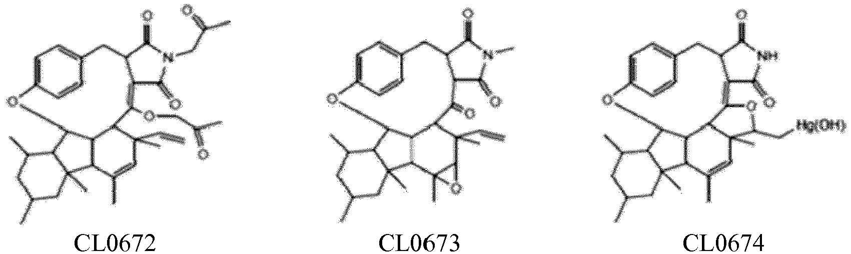

Compound CL0652 was prepared as follows:
13 mg of compound GKK1032 were dissolved in 10 mL of CH₂Cl₂ and 1 mL of CH₃CN. Then two teaspoons of K₂CO₃ were added. After 30 minutes, 2 CH₃I equivalents were added. After 24 hours, TLC showed the presence of some starting material left, so 2 more CH₃I equivalents were added. 4 hours later, another 2 equivalents of CH₃I were added. Finally, the reaction was removed 24 hours later, with a total of 6 equivalents of CH₃I. The solvent was evaporated and the residue dissolved in EtOAc, washed with a saturated aqueous brine solution, dried with anhydrous Na₂SO₄, filtered and evaporated to dryness, affording 11 mg of CL0652.

Compound CL0653 was prepared as follows:
23 mg of compound GKK1032 were dissolved in 25 mL of CH₂Cl₂ and 1.2 N-Bromosuccinimide (NBS) equivalents were added. The reaction was monitored by TLC. After 24 hours, 1 more NBS equivalent was added. Finally, after 48 hours the reaction mixture was washed with a saturated aqueous brine solution, affording 27 mg of reaction mixture that was purified by preparative TLC, eluted with Hexane/EtOAc 6:4, isolating 8 mg of pure CL0653.

Compound CL0661 was prepared as follows:
60 mg of compound GKK1032 were dissolved in dry THF and an excess of NaH was added (under a nitrogen atmosphere). After 72h, the reaction oil was poured onto ice and EtOAc and the THF was evaporated. The residue obtained was washed with a saturated brine solution, affording 51 mg of reaction mixture that was purified by preparative TLC, eluted with Hexane/EtOAc 6:4, isolating 9 mg of pure CL0661 and 20 mg of starting material.

Compounds CL0663 and CL0664 were prepared as follows:
237 mg of compound GKK1032 were dissolved in 20 mL of CH₂Cl₂ and 20 mL of CH₃CN. Then two teaspoons of K₂CO₃ were added. After 30 minutes, 3 equivalents of CH₃I were added. The reaction was monitored by TLC, and finally, after 6 days and a total of 5 CH₃I equivalents, the solvent was evaporated and the residue dissolved in EtOAc, washed with a saturated brine solution, dried with Na₂SO₄ anhydrous, filtered and evaporated to dryness. The reaction mixture (218 mg) was crystallized in CH₃OH, obtaining 120 mg of pure CL0652 and 96 mg of mother liquors. The mother liquors were purified by preparative TLC, eluted with Hexane/EtOAc 8:2, affording 16 mg of CL0652, 12 mg of CL0663 and 10 mg of CL0664.

Compound CL0665 was prepared as follows:
83 mg of compound GKK1032 were dissolved in 40 mL of CH₃OH and cooled to 0 °C with ice. 5 equivalents of NaBH₄ and LiCl respectively were added. After 8 days, the reaction was washed with a saturated brine solution. The reaction mixture was crystallized with CH₃OH, affording 60 mg of a precipitate, that was purified by preparative TLC, eluted with Hexane/EtOAc 7:3. After that, 12 mg of CL0665 were isolated and recrystallized with methanol, to afford 3 mg of pure CL0665.

Compound CL0666 was prepared as follows:
85 mg of compound GKK1032 were dissolved in 20 mL of THF and cooled to 0 °C with ice. 5 equivalents of LiAlH₄ were then added. After 6 days the reaction was poured onto EtOAc and ice. THF was evaporated and the residue washed with a saturated brine solution. The reaction product was further purified by preparative TLC, eluted with Hexane/EtOAc 2:8, affording 18 mg of pure CL0666.

Compound CL0667 was prepared as follows:
48 mg of compound GKK1032 were dissolved in 2 mL of DMF. Two teaspoons of K₂CO₃ were then added. After 30 minutes, 2 equivalents of chloroacetonitrile were added. After 24 hours, 2 more equivalents of chloroacetonitrile were added and stirred for another 24 hours. Then the DMF was evaporated, the crude residue dissolved in EtOAc and washed with a saturated solution of NaCl, affording 55 mg of crude residue that was further purified by preparative TLC eluted with CH₂Cl₂/CH₃OH 98:2, isolating 15 mg of CL0667, that were finally subjected to a silica gel column chromatography, eluted with Hexane/EtOAc 6:4, to get 12 mg of pure CL0667.

Compound CL0669 was prepared as follows:
43 mg of compound GKK1032 were dissolved in 3 mL of DMF and an excess of K₂CO₃ was added. After 30 minutes, 1.5 equivalents of chloroacetonitrile were added. After 5 days and a total of 3 equivalents of chloroacetonitrile, the DMF was evaporated and the reaction mixture dissolved in EtOAc and washed with a saturated brine solution. Then the reaction mixture was taken to dryness and purified by a silica gel column chromatography, eluted with CH₂Cl₂/CH₃OH 92:8, to isolate 34 mg of CL0669, that were further purified by preparative TLC eluted with CH₂Cl₂/CH₃OH 96:4, affording 9 mg of pure CL0669.

Compound CL0670 was prepared as follows:
32 mg of compound GKK1032 were dissolved in 20 mL of CH₂Cl₂ and 1.2 equivalents of metachloroperbenzoic acid were added. After 4 days the reaction mixture was washed with a 5% NaHCO₃ solution, neutralized with a saturated brine solution and taken to dryness. The crude residue was crystallized from CH₃OH, affording 10.5 mg of pure CL0670.

Compounds CL0671 and CL0672 were prepared as follows:
79 mg of compound GKK1032 were dissolved in DMF, and 5 equivalents of chloroacetone and an excess of K₂CO₃ were added. After 4 days DMF was evaporated and the reaction mixture dissolved in EtOAc and washed with a saturated brine solution, to get 100 mg of a complex mixture that was further purified by preparative TLC, eluted with Hexane/EtOAc 6:4, affording 14.8 mg of CL0671 and 12.2 mg of CL0672. Both compounds were finally crystallized in methanol, to get 7 mg of pure CL0671 and 5 mg of pure CL0672.

Compound CL0673 was prepared as follows:
28 mg of compound CL0652 were dissolved in 20 mL of CH₂Cl₂ and 1.2 equivalents of metachloroperbenzoic acid were added. After 24 hours reaction mixture was washed with a 5% NaHCO₃ solution, neutralized with a saturated brine solution and taken to dryness. The crude residue was crystallized from CH₃OH, affording 12.7 mg of pure CL0673.

Compound CL0674 was prepared as follows:
67 mg of compound GKK1032 were dissolved in 15 mL of CH₂Cl₂ and 1.2 equivalents of mercury acetate (II) were added. After 6 days and several additions of mercury acetate (II), the reaction mixture was washed with a saturated aqueous solution of NaCl and evaporated to dryness. The crude residue was further purified by preparative TLC, eluted with Hexane/EtOAc 1:1, to isolate 58 mg of CL0674 that were finally crystallized from methanol, affording 4.2 mg of pure CL0674.

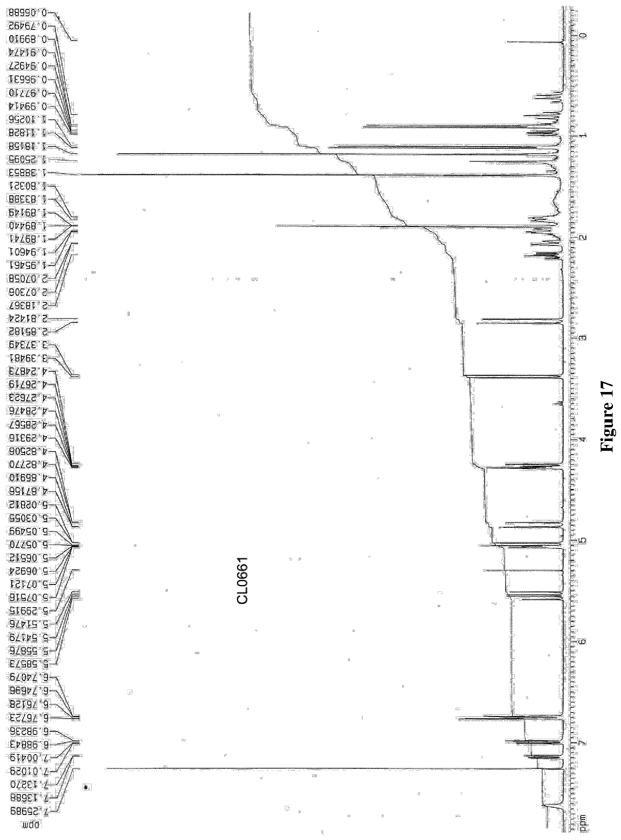

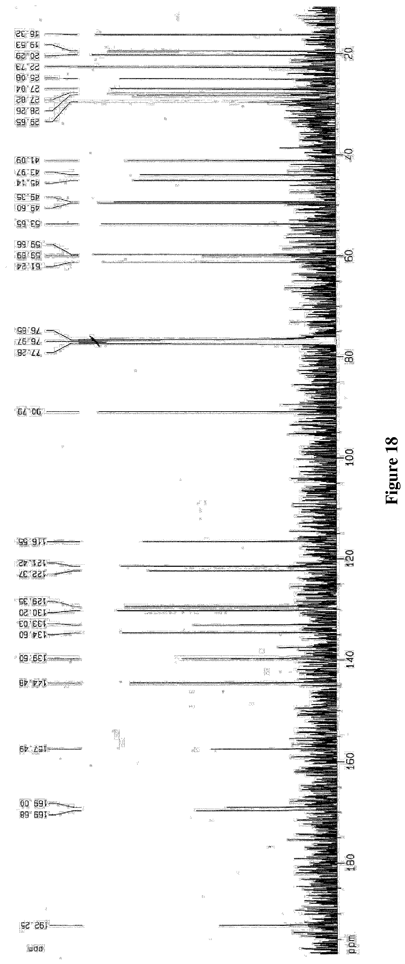

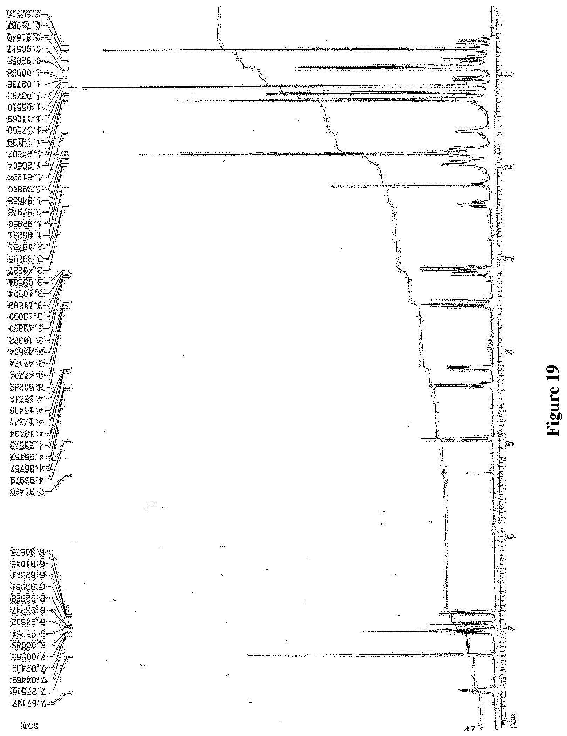

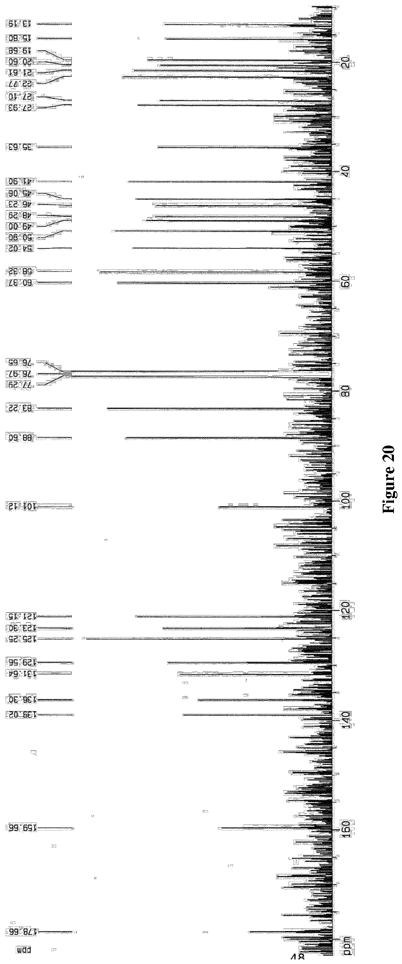

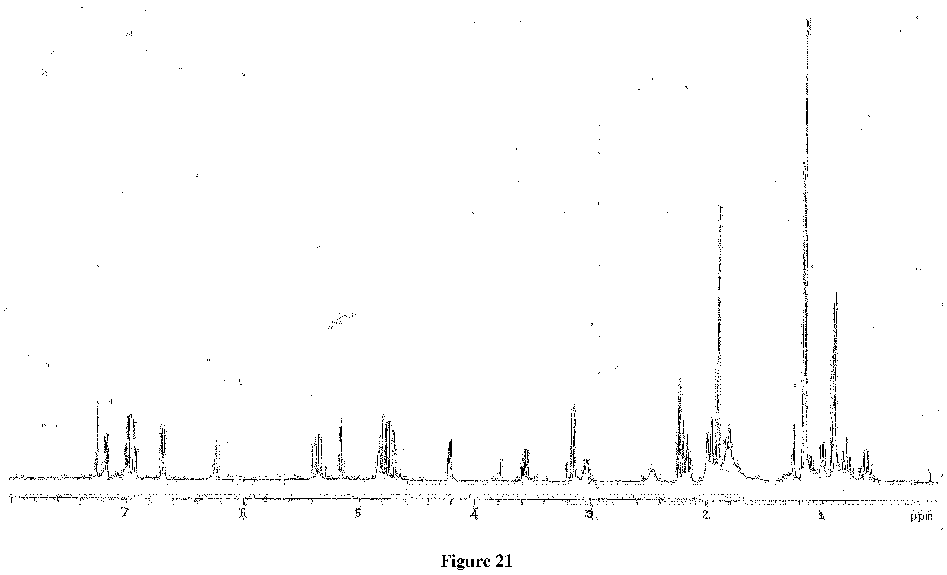

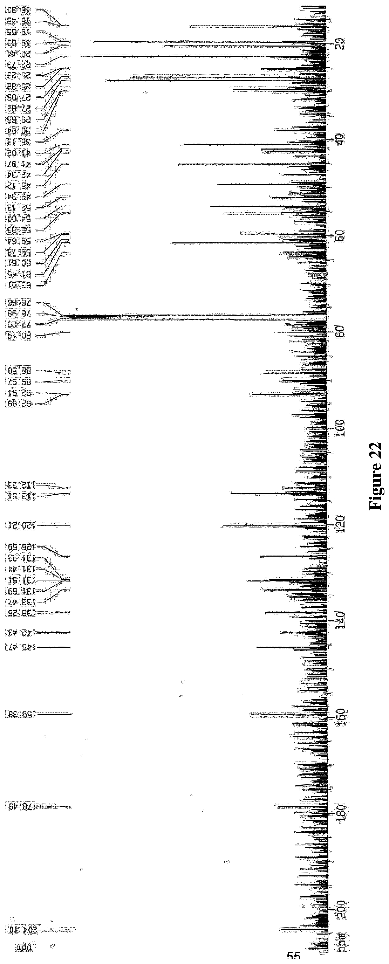

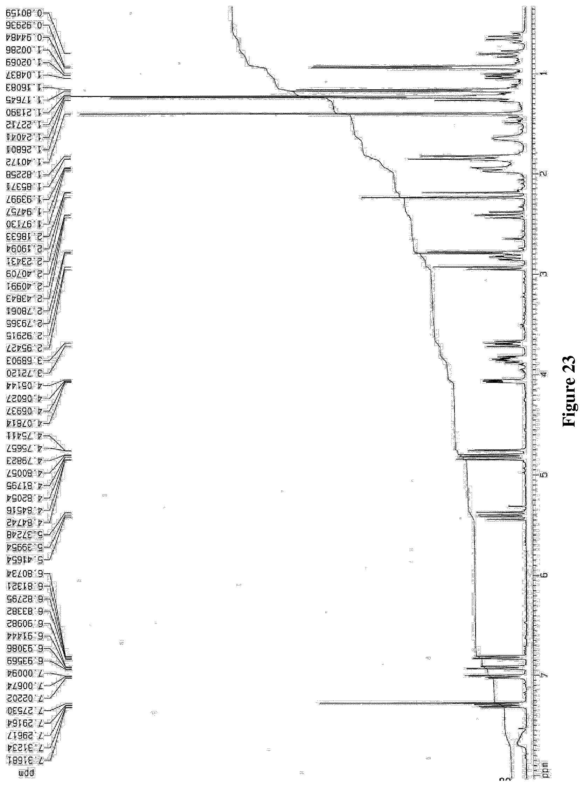

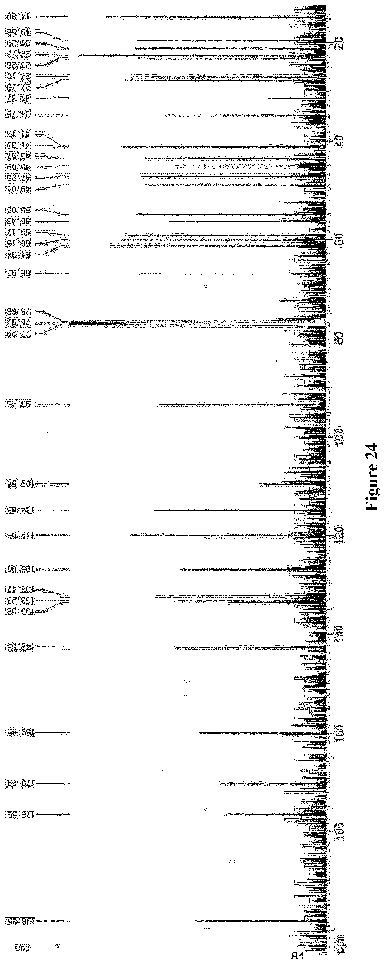

The compounds were identified and characterized by ¹H-NMR, ¹³C-NMR and MS techniques. The NMR spectra (CDCl₃) of compounds CL0661, CL0665, CL0666 and CL0670 are shown in Figures 17 to 24.

### Example 4: Ability of Compound I, GKK1032, and Pyrrocidine A to interfere with reactive oxygen species formation.

Experiments have been performed in rat cortical primary neurons, obtained as was previously described (Herrero-Mendez A. et al., Nat. Cell. Biol. 11:747-752, 2009). After 7 or 8 days in culture, neurons were incubated in the absence or presence of L-buthionine sulfoximine (L-BSO; 100 µM), an inhibitor of glutamate-cysteine ligase hence causing cellular depletion of glutathione, and the mitochondrial complex I inhibitor, rotenone (0.2 µM).

The data are presented as the mean values ± standard deviation of the mean (SEM) from n=6 (i.e., 6 independent wells measured independently). L-BSO and rotenone treatment significantly stimulated the endogenous production of reactive oxygen and nitrogen species (RONS) in neurons, as detected by the release of H₂O₂ using the fluorescent method Amplex© Red hydrogen peroxide/peroxidase assay kit (Invitrogen, catalog number A22188) using a multiwell fluorescence reader (Varioskan, Thermo Scientific).

H₂O₂ was mixed with Compound I, GKK1032, and Pyrrocidine A to assess whether it interfered with the H₂O₂ determination method, resulting in the absence of such interference.

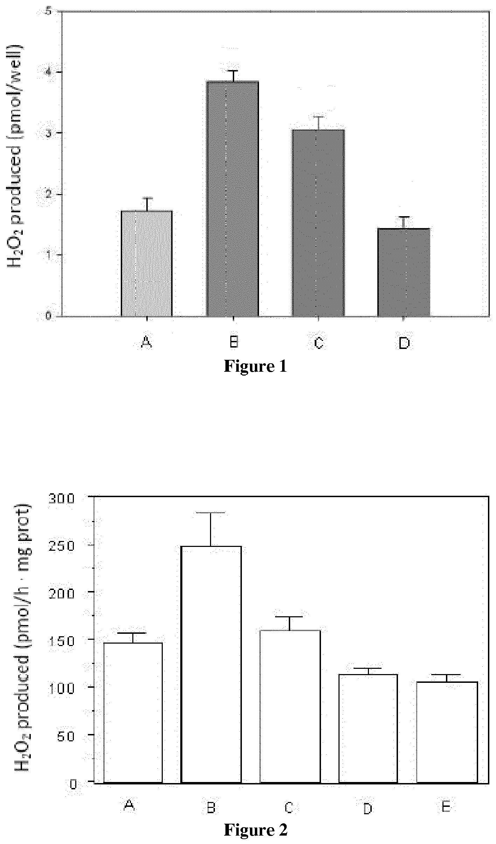

Four different experiments were carried out to investigate the ability of compounds to interfere with endogenous reactive oxygen species formation.
a) Neurons were treated with L-BSO and rotenone during 6 h and then washed twice with phosphate-buffered saline (PBS), after which Compound I was added to the neurons at the concentrations of 1 and 5 µg/ml, and the release of H₂O₂ was immediately recorded during the following 2h. As shown in Figure 1, Compound I dose-dependently decreased the rate of H₂O₂ formation by the neurons.
b) Neurons were incubated with Compound I, or GKK1032, or Pyrrocidine A (1 µg/ml), together with L-BSO and rotenone for 6 h, after which cells were washed twice with PBS and the rate of H₂O₂ formation recorded for the following 2 h. As shown in Figure 2, the pre-treatment of neurons with Compound I, or GKK1032, or Pyrrocidine A, significantly impaired the rate of H₂O₂.
c) Neurons were incubated with Compound I (5 µg/ml) for 72 h (i.e., from day 4 to day 7 in culture), after which cells were washed twice with PBS and further incubated with L-BSO and rotenone for 6 h. After this period, cells were again washed and determined the rate of H₂O₂ production during the next 2 h. As shown in Figure 3, pre-treatment of neurons with Compound I very significantly prevented the L-BSO+rotenone-induced H₂O₂ formation. Compound I also prevented H₂O₂ formation in untreated neurons.
d) Protein concentration was determined in the neurons after 3 or 6 h of incubation with L-BSO and rotenone in the presence of different concentrations of Compound I (0.1, 1 or 5 µg/ml). As shown in Figure 4, the incubation of neurons with L-BSO plus rotenone slightly decreased the protein concentration remaining in the wells, suggesting a low but measurable level of neuronal death. In contrast, the presence of Compound I dose-dependently prevented such decrease in protein concentrations as from 0.1 µg/ml (6 h). Thus, Compound I is revealed as an anti-neurotoxic and pro-surviving compound.

### Example 5: Compounds of formula (I) in preventing H₂O₂ formation.

Experiments were performed in rat cortical primary neurons, obtained as was previously described (Herrero-Mendez A. et al., Nat. Cell. Biol. 11:747-752, 2009). After 7 or 8 days in B27-MAO culture, neurons (from C57BL6/J mice) were incubated in the presence of N-methyl-D-aspartate (NMDA, a H₂O₂ production promoter; 100 µM) for 10 minutes, followed by a PBS washing step and submitted to a 24-hour B27 incubation step in the absence (DMSO) or presence of increasing amounts of Compound I, GKK1032, and Pyrrocidine B (0.1 µM, 1 µM and 5 µM). B27-MAO stands for Neurobasal medium, supplemented with B27 supplement in the absence of antioxidants (minus antioxidants, MAO).

The release of H₂O₂ was measured using the fluorescent method Amplex© Red hydrogen peroxide/peroxidase assay kit (Invitrogen, catalog number A22188) using a multiwell fluorescence reader (Varioskan, Thermo Scientific).

Neuronal apoptosis was determined by measuring caspase-3 activity (Veas-Perez de Tudela et al. Sci Rep 5:18180; doi:10.1038/srep18180 (2015)).

The data are presented as the mean values ± standard deviation of the mean from n=3

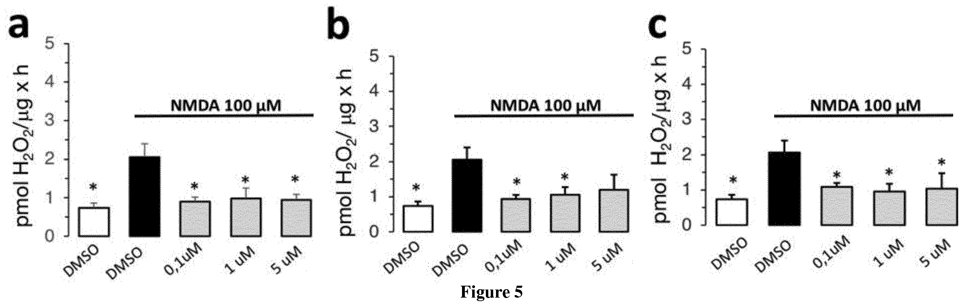

As shown in Figure 5, Compound I, GKK1032, and Pyrrocidine B were successful in preventing H₂O₂ from being produced in the neuron cells.

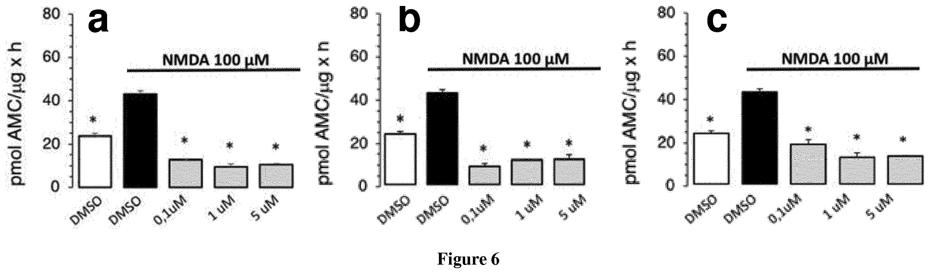

In addition, Compound I, GKK1032, and Pyrrocidine B were effective in protecting neuron cells against apoptosis, as shown in Figure 6.

### Example 6: Compounds of formula (I) in preventing neuronal apoptosis.

Experiments were performed in rat cortical primary neurons, obtained as was previously described (Herrero-Mendez A. et al., Nat. Cell. Biol. 11:747-752, 2009). After 7 or 8 days in B27-MAO culture, neurons (from C57BL6/J mice) were incubated with β-amyloid (10 µM, 24 h) in the absence (DMSO) or presence of increasing concentration of compounds of formula (I): compound I and compounds CL0661, CL0665, CL0666 and CL0670 (concentrations of 0.1 µM, 1 µM, 5 µM and 10 µM). Treatment of neuronal cells with β-amyloid was chosen to mimic, *in vitro,* the formation of amyloid plaques found in the brains of Alzheimer patients. B27-MAO stands for Neurobasal medium, supplemented with B27 supplement in the absence of antioxidants (minus antioxidants, MAO).

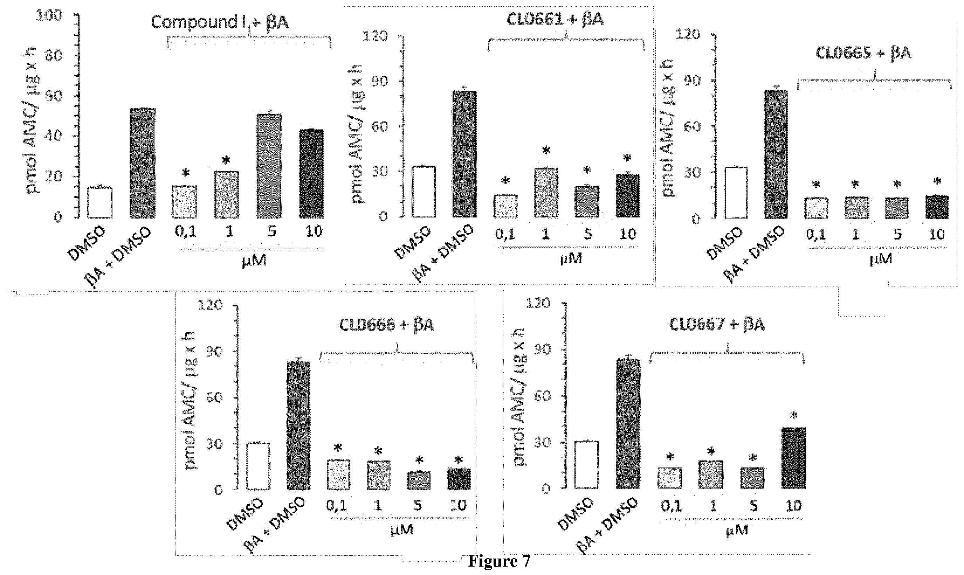

The neuronal apoptosis from the 24-hour incubation with β-amyloid was measured by measuring the active caspase-3 and the results are shown in Figure 7.

The tested compounds all showed a clear neuroprotector effect at the tested concentrations.

### Example 7: Maximum Tolerated Dose and Pharmacokinetics of Compound I.

The Maximum Tolerated Dose (MTD) and Pharmacokinetics (PK) of compound I were determined after intraperitoneal administration to female C57BL/6 (∼25 g) mice. The injection comprised a 150 mg/Kg dose of compound I in Creomophor-EtOH-H₂O (1-0.5-8.5) and samples were collected at t = 0 min, 5 min, 15 min, 30 min, 1 hour, 4 hours, 10 hours, 24 hours and 48 hours.

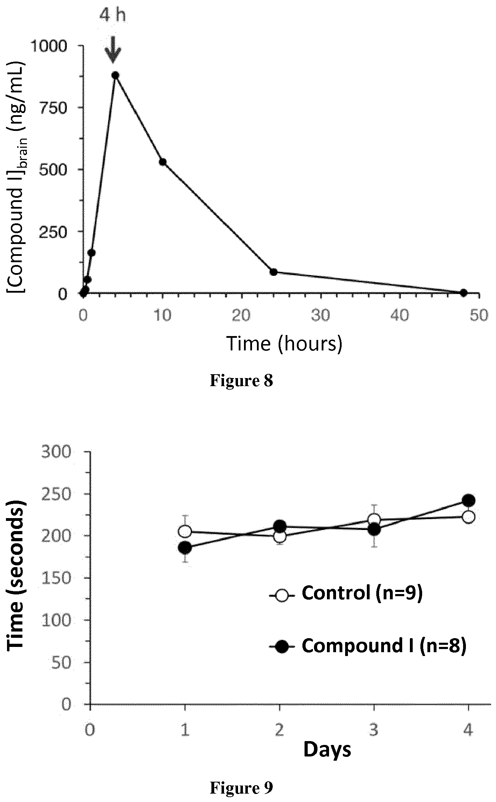

The results are represented in Figure 8 and show that Compound I successfully crosses the blood-brain barrier reaching concentrations as high as 850 ng/mL at 4 hours after injection.

### Example 8: Compound I and its effect under normal conditions to mice motor coordination, fear/anxiety and short-term memory.

Experiments were conducted to evaluate if the intraperitoneal administration of Compound I to mice would have any effect in the animal's motor coordination, fear/anxiety and short-term memory behaviour. The motivation for this experiment is to determine if, under normal conditions, the injection of compound I would affect the behaviour of 8 weeks-old C57BL6/J male mice, *in vivo.* Mice were separated in group I (vehicle injection only) and group II (compound I injection). The mice were injected with a once-daily dose of 150 mg/kg for 3 days. The tests consisted in the rotarod performance test (at day 1, 2, 3 and 4), the open field test (at day 2 and 3) and the novel object recognition test (at day 4 only).

Figure 9 shows that compound I had no effect in the motor coordination of the tested animals.

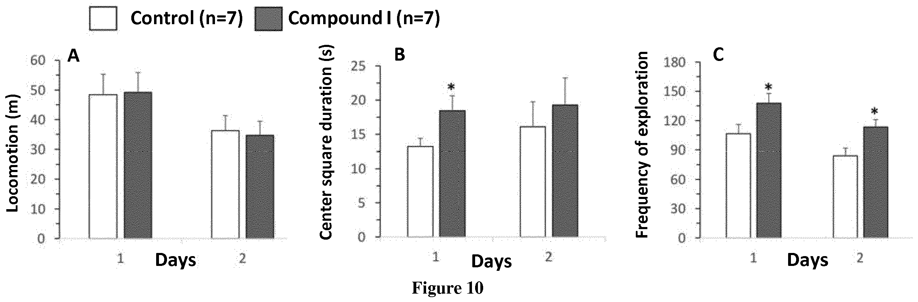

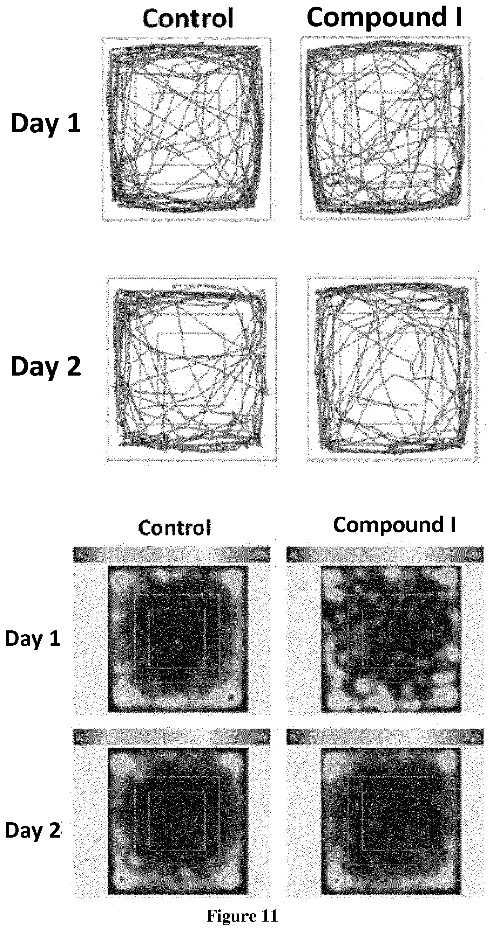

Figures 10 and 11 show the open field test results, which provide indication of the animal's behavioural changes regarding fear and/or anxiety. Surprisingly, Compound I increased the time that the animals spent in central square of the arena as well as an increase in the rearing (frequency with which the rodent stands on its hind legs in the central square). Compound I further increased the number of line crossings (i.e., the frequency with which the rodent crosses grid lines). The results of this experiment are evidence that the animals had an increased exploratory behaviour and that Compound I had a disinhibition effect by reducing fear and/or by reducing anxiety levels.

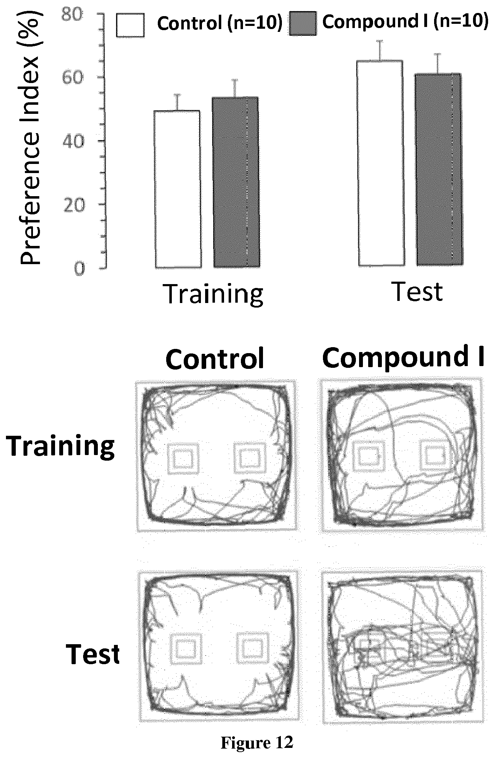

Figure 12 shows the Novel Object Recognition test. Results showed that the administration of compound I did not alter the rodent's short-term memory.

### Example 9: Compound I and its effect in protecting neurons from Huntington's disease in a rat model.

A chemical model of Huntington's disease was conducted to evaluate the capacity of compound I in protecting neurons. This is achieved by intraperitoneal injection of 3-nitropropionic acid which induces neuronal oxidative stress by inhibiting the succinate dehydrogenase enzyme.

8-weeks old C57BL6/J male mice were divided into 4 groups.

Mice in group I (4 mice) were injected with Compound I (once-daily dose of 150 mg/kg for 2 days).

Mice in group II (5 mice) were injected with Compound I (once-daily dose of 150 mg/kg for 2 days) and with 3-nitropropionic acid (twice daily dose of 50 mg/kg for 4 days). In the case of group II, 3-nitropropionic acid was injected two hours after injection with Compound I. Mice in this group were thus treated with Compound I during the first half of the 4-day experiment.

Mice in group III (5 mice) were injected with 3-nitropropionic acid (twice daily dose of 50 mg/kg for 4 days).

Mice in group IV (6 mice) were injected with vehicle only.

The mice were submitted to the rotarod test once-daily, 2 hours after the injection(s). At day 4, the mice were submitted to the rotarod test at the same time of the day as for days 1-3, and after the test, samples were collected.

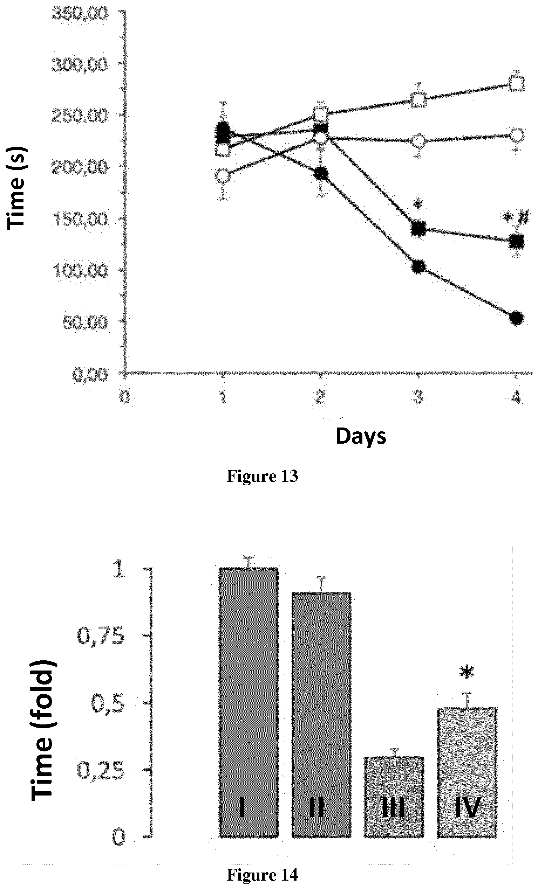

Figure 13 shows the rotarod test results for groups I (□), II (■), III (●) and IV (○). Motor coordination was significantly affected for those mice pertaining to group III (treated only with the oxidative stress inducer 3-nitropropionic acid) since the first day of experiment. This result is in agreement with a Huntington's disease scenario. Mice in group II showed normal behaviour during the first half of the 4-day experiment, but once treatment with Compound I was stopped after day 2, a significant decrease in motor abilities was observed thus providing evidence of the beneficial effect provided by Compound I.

### Example 10: Ability of Compound I in protecting neurons from stroke in a rat model.

Middle Cerebral Artery occlusion involves temporarily or permanently restricting MCA blood flow to the cortex and striatum. The cerebral ischemic model of stroke is commonly used to assess ischemic injury in the brain. Occlusion of the middle cerebral artery (MCA) leads to a reduction of cerebral blood flow in both the striatum and the cortex. It is a highly reproducible model that results in brain damages similar to those occurring in human stroke.

Compound was administered intravenously at a dose of 10 mg/kg.

8-week old C57BL6/J mice were divided into 4 groups of 7 mice each:
Group I - surgery without occlusion
Group II - surgery without occlusion and administration of Compound I
Group III - Middle Cerebral Artery occlusion
Group IV - Middle Cerebral Artery occlusion and administration of Compound I.

All mice were firstly trained in the rotarod for 3 consecutive days. After this period, the mice were put to sleep with sevoflurane and submitted to surgery. Occlusion was performed by maintain the surgical filament occluding the middle cerebral artery for 30 minutes. Once removed, compound I was injected at the jugular vein. 24 hours after reanimation, the animals were submitted to the rotarod test.

The results are shown in Figure 14, where it is observed that while mice pertaining to groups I and II showed similar motor coordination, those mice pertaining to group III, which were submitted to the occlusion procedure (stroke), suffered from dramatic (∼75%) motor coordination impairment. The immediate injection of Compound I to mice of group IV (stroke) greatly improved the recovery of the animals, as shown in Figure 14 (IV).

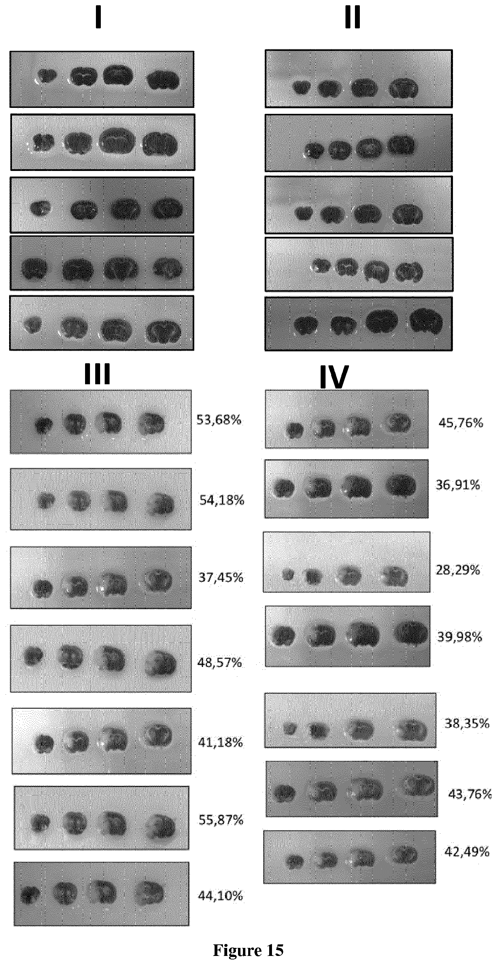

To compare the neuroprotective effect caused by Compound I to the motor coordination of mice and the cerebral damage caused by the chirurgical procedure, the animals were sacrificed and the stroke size measured by visual inspection after treatment with tetrazolium chloride. Figure 15 shows that Compound I has no effect in the morphology (comparison between images of group I and II). Figure 15 further shows that for mice of group III, the percentage averaged stroke volume was of 47.9% against a percentage averaged stroke volume for mice of group IV (treated with Compound I) of 39.4%. The data confirms that Compound I is an excellent neuroprotector agent against stroke.

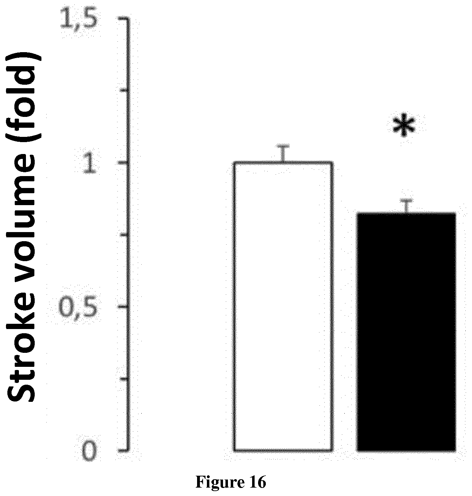

Figure 16 shows that Compound I is capable of protecting roughly 18% of the stroke volume caused by the Middle Cerebral Artery occlusion.

## Claims

1. Compound of formula: 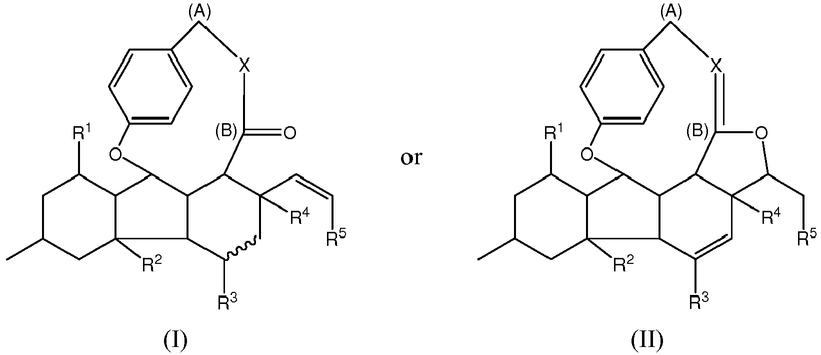 wherein
the wavy bond ∼ is either a double bond or the epoxide, 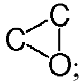
R¹, R², R³ and R⁴ are each independently selected from the group consisting of hydrogen and substituted or unsubstituted C₁-C₆ alkyl;
R⁵ is selected from the group consisting of hydrogen, substituted or unsubstituted C₁-C₆ alkyl, or halogen;
X denotes a group represented by formulas (III) to (XII), 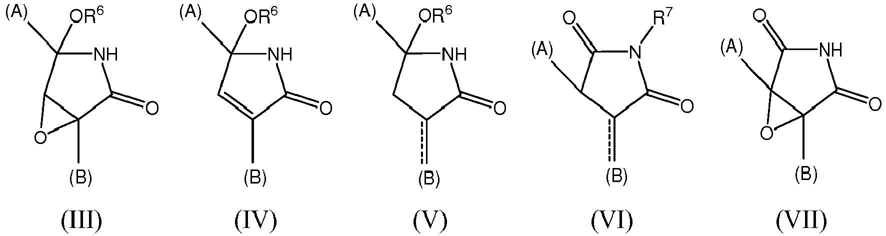 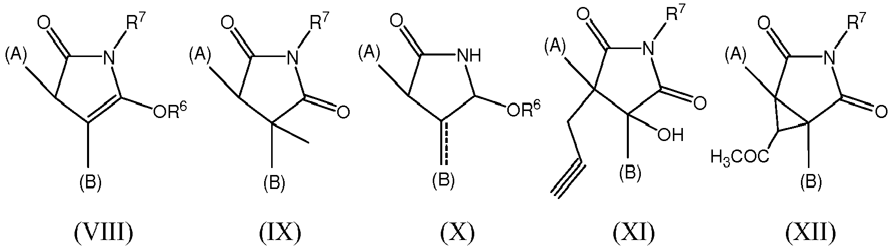
wherein
the dashed bond represents an optional double bond which, when present, is the double bond between X and (B) in the compound of formula (II);
R⁶ is selected from the group consisting of hydrogen, substituted or unsubstituted acyl, substituted or unsubstituted alkyl, substituted or unsubstituted alkenyl, substituted or unsubstituted alkynyl, substituted or unsubstituted aryl, substituted or unsubstituted aralkyl, -CONH₂, alkali metal, and sugar; and
R⁷ is selected from the group consisting of hydrogen, substituted or unsubstituted C₁-C₆ alkyl, substituted or unsubstituted C₁-C₆ alkynyl, nitrile, and -CH₂-CO-CH₃;
or pharmaceutically acceptable salts, solvates, tautomers, or stereoisomers thereof, for use in the treatment of diseases associated with oxidative stress.

2. Compound for use according to claim 1, wherein the disease is selected from neurodegenerative diseases, stroke, mitochondriopathies with neuromuscular affection, cardiovascular diseases, pancreatitis, and cirrhosis.

3. Compound for use according to claim 2, wherein the neurodegenerative disease is selected from Parkinson's disease, Alzheimer's disease, senile dementia, Huntington's disease, multiple sclerosis and amyotrophic lateral sclerosis.

4. Compound for use according to any one of claims 1 to 3, wherein R¹, R², R³, R⁴ and R⁵ are each independently selected from the group consisting of hydrogen and methyl.

5. Compound for use according to any one of claims 1 to 4, wherein R⁶ is selected from the group consisting of hydrogen, substituted or unsubstituted C₂-C₁₂ acyl, substituted or unsubstituted C₁-C₆ alkyl, substituted or unsubstituted C₂-C₆ alkenyl, substituted or unsubstituted C₂-C₆ alkynyl, substituted or unsubstituted C₆-C₁₀ aryl, substituted or unsubstituted (C₆-C₁₀)aryl(C₁-C₆)alkyl, -CONH₂, alkali metal, and sugar.

6. Compound for use according to claim 5, wherein R⁶ is selected from the group consisting of hydrogen, substituted or unsubstituted acetyl, substituted or unsubstituted methyl, ethyl, phenyl and substituted or unsubstituted benzyl.

7. Compound for use according to any one of claims 1 to 6, wherein the compound of formula (I) or (II) is selected from: 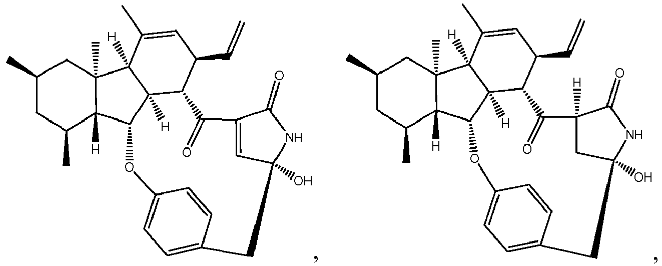 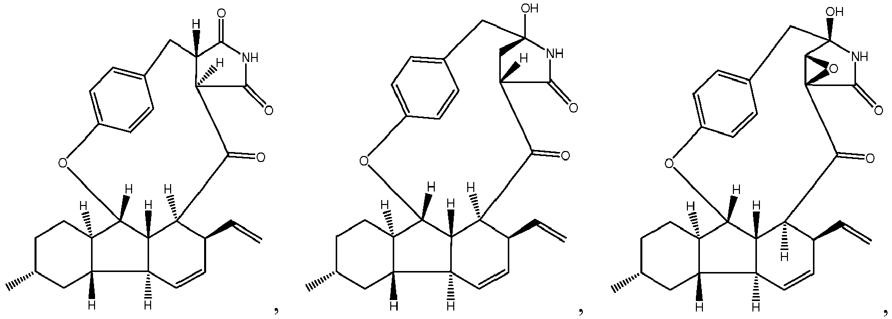 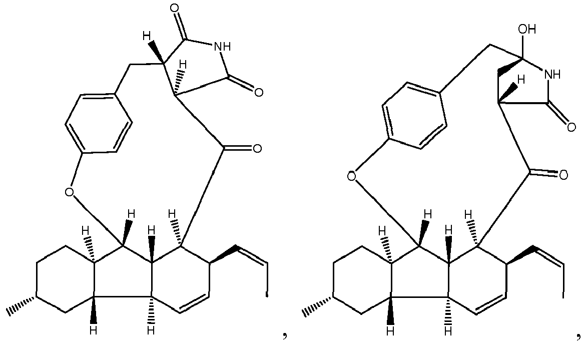 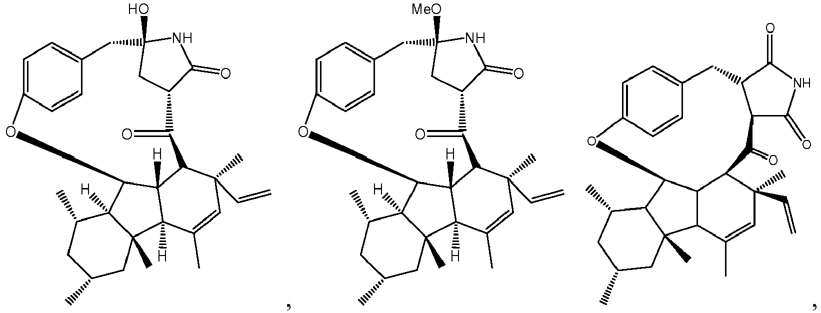 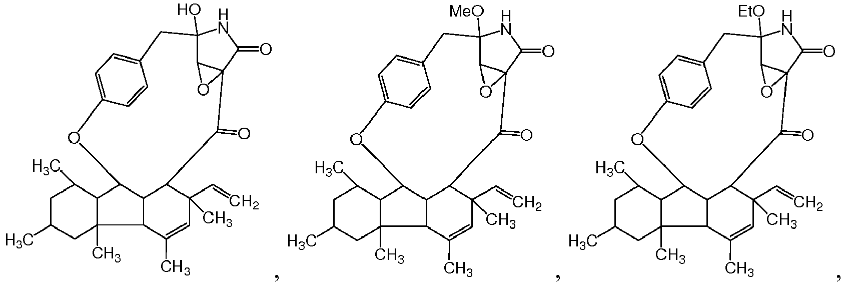 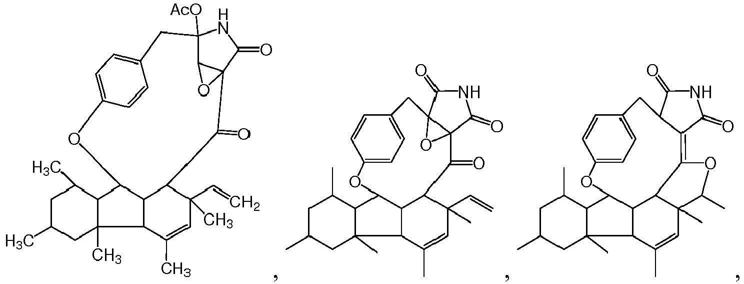 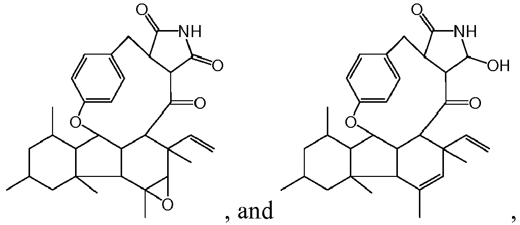 or pharmaceutically acceptable salts, solvates, tautomers, or stereoisomers thereof.

8. A compound of formula : 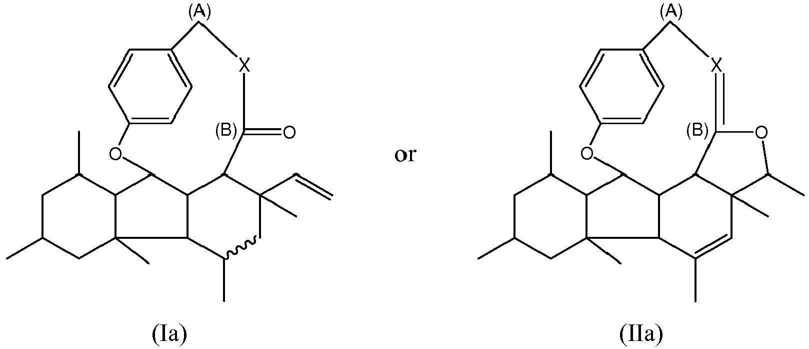 wherein
the wavy bond ∼ is either a double bond or the epoxide, 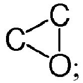 X denotes a group represented by formulas, 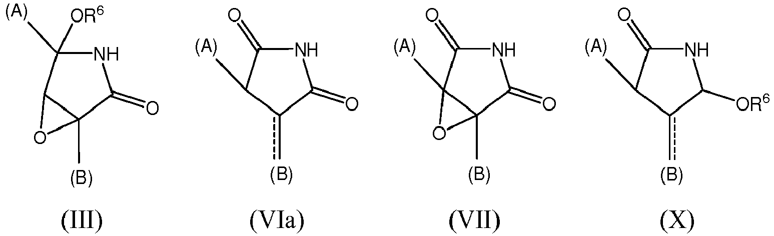 wherein
the dashed bond represents an optional double bond which, when present, is the double bond between X and (B) in the compound of formula (II);
wherein R⁶ is selected from the group consisting of hydrogen, substituted or unsubstituted acyl, substituted or unsubstituted alkyl, substituted or unsubstituted alkenyl, substituted or unsubstituted alkynyl, substituted or unsubstituted aryl, substituted or unsubstituted aralkyl, -CONH₂, alkali metal, and sugar,
or a pharmaceutically acceptable salt, tautomer, solvate or stereoisomer thereof,
with the proviso that compound GKK1032, of formula 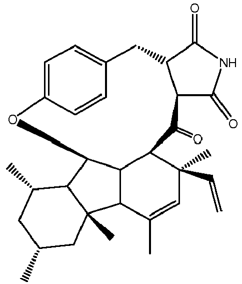 is excluded.

9. Compound according to claim 8, wherein R⁶ is selected from the group consisting of hydrogen, substituted or unsubstituted C₂-C₁₂ acyl, substituted or unsubstituted C₁-C₆ alkyl, substituted or unsubstituted C₂-C₆ alkenyl, substituted or unsubstituted C₂-C₆ alkynyl, substituted or unsubstituted C₆-C₁₀ aryl, substituted or unsubstituted (C₆-C₁₀)aryl(C₁-C₆)alkyl, -CONH₂, alkali metal, and sugar.

10. Compound according to claim 9, which is selected from: 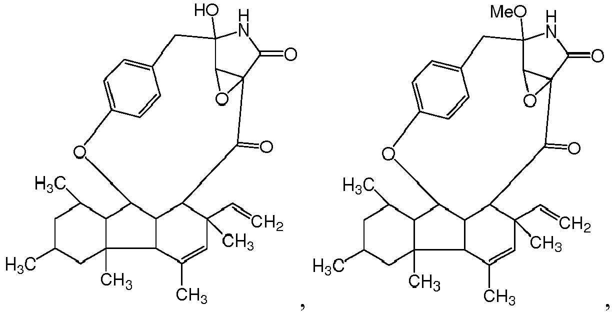 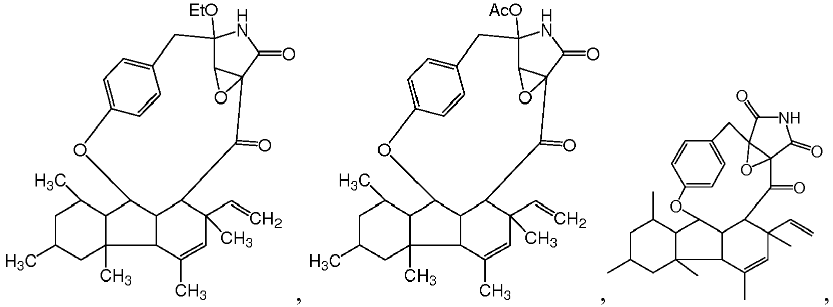 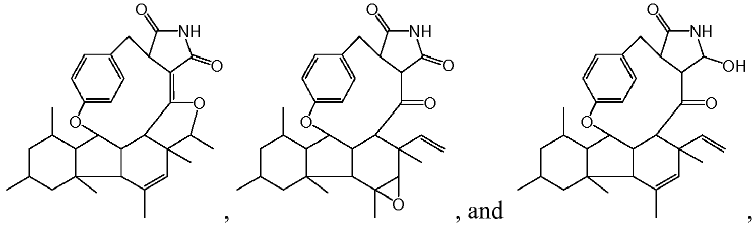 or pharmaceutically acceptable salts, solvates, tautomers, or stereoisomers thereof.

11. A process for preparing a compound as defined in any one of claims 8 to 10 which comprises cultivating the strain HT-09B-VENT-WQ002, available under accession number CECT 20769 from the Colección Española de Cultivos Tipo at Valencia University, Spain, in an aqueous nutrient medium, isolating and purifying the resulting compound from the cultured broth and, optionally, modifying this compound to obtain a compound as defined in any of claims 8 to 10.

12. A pharmaceutical composition comprising a compound as defined in any of claims 8 to 10 or a pharmaceutically acceptable salt, solvate, tautomer or stereoisomer thereof, and a pharmaceutically acceptable carrier.

13. A compound as defined in any one of claims 8 to 10 or a pharmaceutically acceptable salt, solvate, tautomer or stereoisomer thereof, for use as a medicament.
